(19) Europäisches Patentamt
European Patent Office
Office européen des brevets

(11) **EP 2 634 748 B1**

(12) # EUROPÄISCHE PATENTSCHRIFT

(45) Veröffentlichungstag und Bekanntmachung des Hinweises auf die Patenterteilung:
**12.06.2019 Patentblatt 2019/24**

(51) Int Cl.:
***G06T 3/00*** *(2006.01)*

(21) Anmeldenummer: **12181000.6**

(22) Anmeldetag: **20.08.2012**

(54) **Bilddatenbestimmungsverfahren**

Image data calculation method

Procédé de détermination de données d'image

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.02.2012 EP 12157329**
**01.03.2012 EP 12001379**

(43) Veröffentlichungstag der Anmeldung:
**04.09.2013 Patentblatt 2013/36**

(73) Patentinhaber: **Siemens Healthcare GmbH**
**91052 Erlangen (DE)**

(72) Erfinder:
• **Jerebko, Anna**
**91052 Erlangen (DE)**
• **Kelm, Michael**
**91052 Erlangen (DE)**
• **Sühling, Michael**
**91052 Erlangen (DE)**
• **Wels, Michael**
**96047 Bamberg (DE)**

(56) Entgegenhaltungen:
• **STELIOS K KYRIACOU * ET AL: "<tex> \input /arbortext/local/styles/bgtex.sty</tex></maths>Nonlinear Elastic Registration of Brain Images with Tumor Pathology Using a Biomechanical Model", IEEE TRANSACTIONS ON MEDICAL IMAGING, IEEE SERVICE CENTER, PISCATAWAY, NJ, US, Bd. 18, Nr. 7, 1. Juli 1999 (1999-07-01), XP011035875, ISSN: 0278-0062**
• **YONG ZHANG ET AL: "3D Finite Element Modeling of Nonrigid Breast Deformation for Feature Registration in -ray and MR Images", APPLICATIONS OF COMPUTER VISION, 2007. WACV '07. IEEE WORKSHOP ON, IEEE, PI, 1. Februar 2007 (2007-02-01), Seiten 38-38, XP031055147, ISBN: 978-0-7695-2794-9**
• **YAN QIU ET AL: "Correspondence recovery in 2-view mammography", BIOMEDICAL IMAGING: MACRO TO NANO, 2004. IEEE INTERNATIONAL SYMPOSIUM ON ARLINGTON,VA, USA APRIL 15-18, 2004, PISCATAWAY, NJ, USA,IEEE, 15. April 2004 (2004-04-15), Seiten 197-200, XP010773831, DOI: 10.1109/ISBI.2004.1398508 ISBN: 978-0-7803-8389-0**

**Beschreibung**

Die Erfindung betrifft ein Verfahren in der radiologischen Bildgebung zur Bestimmung einer zweiten Form von Bilddaten aus einer ersten Form von Bilddaten eines Untersuchungsobjekts. Darüber hinaus betrifft die Erfindung eine Bildbearbeitungsstation in der radiologischen Bildgebung zur Bestimmung einer zweiten Form von Bilddaten aus einer ersten Form von Bilddaten sowie eine Bildgebungseinrichtung.

Bildgebende Systeme der Medizintechnik nehmen heute eine bedeutende Rolle bei der Untersuchung von Patienten ein. Die von den bildgebenden Systemen erzeugten Darstellungen der inneren Organe und Strukturen des Patienten werden zu vorbeugenden Untersuchungen (Screening), zur Gewebeentnahme (Biopsie), der Diagnose von Krankheitsursachen, zur Planung von Operationen, bei der Durchführung von Operationen oder auch zur Vorbereitung von therapeutischen Maßnahmen angewandt. Beispiele für solche bildgebenden Systeme sind Ultraschallsysteme, Röntgengeräte, Röntgen-Computertomographie(CT)-Systeme, Positronen-Emissionstomographie(PET)-Systeme, Single-Photon-Emissionstomographie(SPECT)-Systeme oder Magnetresonanz(MR)-Systeme.

Die einzelnen bildgebenden Systeme unterscheiden sich dabei zum Einen dadurch, welche Körperbereiche des Patienten in der jeweiligen Anwendung dargestellt werden sollen. So eignen sich Geräte und Systeme, die auf Röntgenstrahlen basieren, eher für die Darstellung von Knochen und knochenartigen Strukturen, während MR-Systeme eher für die Darstellung von Muskel- oder Fettgewebe und der inneren Organe eingesetzt werden. Zum Anderen spielen die Untersuchungszeiten bei der Bildgebung und die Anschaffungskosten des bildgebenden Systems und die aus Beiden resultierenden Gesamtkosten einer Bildgebung eine wesentliche Rolle. Entsprechend werden kostengünstigere Bildgebungssysteme und Bildgebungsverfahren üblicherweise bei Routineuntersuchungen, wie der vorbeugenden Krebsuntersuchung, eingesetzt, da diese regelmäßig an einer großen Zahl von Patienten durchzuführen sind. Aufwändigere und kostenintensivere Verfahren, die meist detaillierte, dreidimensionale Bilddaten bereitstellen, werden dagegen oft nur dann angewandt, wenn vorbeugende Untersuchungen auf mögliche Erkrankungen hinweisen oder operative Eingriffe zwingend notwendig werden. Ein Einsatz der kostenintensiven bildgebenden Verfahren bei Routineuntersuchungen dagegen ist häufig aus Kostengründen nicht möglich. Darüber hinaus sind die kostenaufwändigen genaueren Bildgebungssysteme vielfach am Ort des Patienten oder zeitlich nicht verfügbar.

Ein weiteres Problem der medizinischen Bildgebung ergibt sich dadurch, dass während einer Bilddatenaufnahme der betreffende Körperbereich des Patienten sich in einer bestimmten Position befinden muss, beziehungsweise auch durch zusätzliche medizinische Apparaturen in eine bestimme Position gebracht oder in einer Position fixiert wird, welche sich für die Bildgebung besonders eignet, insbesondere um Bewegungen des Körperbereichs während der Bilddatenaufnahme zu vermeiden, oder um die Bildqualität durch Standardisierung der Aufnahmesituation und Reduzierung des durchstrahlten Körpervolumens zu erhöhen.

Bei einem anschließenden medizinischen Eingriff an diesem Patienten dagegen kann sich der betreffende Körperbereich des Patienten dann in einer, mitunter wesentlich anderen Position befinden. Insbesondere wird der medizinische Eingriff oft ohne die genannten medizinischen Apparaturen stattfinden, da diese das medizinische Fachpersonal bei der Durchführung des Eingriffs behindern könnten. Das heißt nun aber, dass die Bilddaten des Körperbereich nicht dem Zustand des Körperbereichs während des medizinischen Eingriffs entsprechen, so dass das medizinische Fachpersonal die Bilddaten nur bedingt als Grundlage für die Durchführung des Eingriffs heranziehen kann, beziehungsweise, dass das Risiko besteht, dass der Eingriff in nicht optimaler Weise erfolgt, da eine räumliche Zuordnung der zu operierenden Abschnitte im betroffenen Körperbereich den genannten Abweichungen unterliegen kann.

Es besteht daher ein Bedarf an Verfahren und Einrichtungen in der radiologischen Bildgebung, mit denen sich Bilddaten, die in einer bestimmten Form erzeugt wurden, beispielsweise mit einem kostengünstigen Bildgebungssystem oder in einer bestimmten Positionierung des interessierenden Körperbereichs, in Bilddaten einer weiteren Form umwandeln lassen, beispielsweise in Bilddaten, die sich für einen medizinischen Eingriff eignen. Zur Lösung diese Problems werden in der medizinischen Praxis u.a. auf Basis der Bilddaten Handskizzen mit präoperativen Markierungen durch einen Radiologen angefertigt, die einem Chirurgen Hinweise darauf geben, an welchen Stellen des betreffenden Körperbereichs ein medizinischer Eingriff erfolgen sollte. Es ist aber offensichtlich, dass ein derartiges Vorgehen fehleranfällig ist und damit mit Risiken für den Patienten verbunden sein kann.

Darüber hinaus sind aus G. Schie et al., "Correlating locations in ipsilateral breast tomosynthesis views using an analytical hemispherical compression model", Phys. Med. Biol. 56 (2011) erste Verfahren bekannt, mit denen sich bestimmte Regionen in Bilddaten eines Körperbereichs, der durch eine äußere Apparatur komprimiert wurde, auf Bilddaten abbilden lassen, die unter einem anderen Bildgebungswinkel mit einer abweichenden Komprimierung aufgenommen wurden. Dazu werden bei Schie et al. für den betreffenden Körperbereich spezifische biomechanische Annahmen getroffen und spezifische biomechanische Parameter bestimmt, um die genannte Abbildung zu ermöglichen. Weiterhin werden in Stelios K. Kyriacou et al: "Nonlinear Elastic Registration of Brain Images with Tumor Pathology Using a Biomechanical Model", IEEE Transactions on Medical Imaging, Bd. 18, Nr. 7, Juli 1999, Verfahren beschrieben, mit denen Gehirnatlanten auf zweidimensionale radiologische Bilddaten von Patienten abgebildet werden können, welche unter einer Gehirntumor-Erkrankung und einer darau resultierenden mechanischen Verformung des Gehirns leiden.

Dazu werden dort biomechanische Eigenschaften des Gehirns zugrunde gelegt, um Bilddaten zu erzeugen, die einem Gehirn vor einer Verforumung durch die Tumor-Erkrankung entsprechen. Diese Verfahren sind damit aber auf den jeweiligen Körperbereich limitiert und können nicht ohne weiteren Aufwand auf andere Körperbereiche mit abweichenden biomechanischen Eigenschaften übertragen werden.

Es ist daher die Aufgabe der vorliegenden Erfindung, ein Verfahren und eine Einrichtung anzugeben, welche aus einer ersten Form von radiologischen Bilddaten eines Untersuchungsobjekts Bilddaten in einer geeigneten zweiten Form bestimmt und damit die genannten Probleme löst und die Einschränkungen der bekannten Verfahren vermeidet.

Diese Aufgabe wird durch ein Verfahren nach Anspruch 1, eine Bildbearbeitungsstation nach Anspruch 13, eine Zielobjektbestimmungseinrichtung nach Anspruch 14 und ein Computerprogrammprodukt nach Anspruch 16 gelöst.

Das erfindungsgemäße Verfahren in der radiologischen Bildgebung zur Bestimmung einer zweiten Form von Bilddaten aus einer ersten Form von Bilddaten eines Untersuchungsobjekts umfasst einen ersten Schritt, bei dem in den Bilddaten der ersten Form ein Satz einer definierten Mehrzahl K an Eingangsbildpunkten bestimmt wird. Mit "Mehrzahl" wird dabei hier und im Weiteren eine positive natürliche Zahl größer als Eins bezeichnet. Der Begriff "Untersuchungsobjekt" bzw. "Patient" steht hier und im Folgenden für einen in medizinischer Behandlung befindlichen Menschen oder ein in medizinischer Behandlung befindliches Tier. Dabei sind auch Untersuchungsobjekte mit eingeschlossen, welche keine Erkrankung aufweisen, also auch Menschen, bei denen Bilddaten zur Vorbeugung, z.B. bei einem präventiven Screening zur Krebs-Vorsorge, erzeugt werden. Im Weiteren werden die Begriffe "Untersuchungsobjekt" und "Patient" gleichbedeutend und ohne Einschränkung der Erfindung verwendet. Außerdem wird in der Erfindung im Allgemeinen nicht zwischen weiblichen und männlichen Patienten unterschieden, sondern weitgehend einheitlich der männliche Begriff "Patient" verwendet, auch wenn das Verfahren ebenso an Patientinnen einsetzbar ist.

Die Bilddaten der ersten Form können durch eine Messung oder Bilddatenaufnahme mit einem System der radiologischen Bildgebung erzeugt worden sein. Beispielsweise kann es sich bei den Bilddaten der ersten Form um ein zweidimensionales Bild des Körperbereichs handeln, das mit einer in der medizinischen Praxis üblichen Röntgeneinrichtung aufgenommen wurde. Ebenso sind dreidimensionale Aufnahmeverfahren zur Erzeugung der Bilddaten der ersten Form denkbar, also beispielsweise Verfahren mit CT-, PET- oder MR-Systemen. Zusätzlich sind weitere Ausprägungen von Bilddaten denkbar, beispielsweise dreidimensionale Bilddaten, die mit Hilfe eines sog. 3D-Tomosynthese-Verfahrens aus mehreren zweidimensionalen Bilddatenaufnahmen gewonnen wurden. Entsprechend kann es sich bei dem Satz an Eingangsbildpunkten um einen Satz mit zweidimensionalen Bildpunkten oder dreidimensionalen Bildpunkten handeln, beispielsweise einen Vektor, der eine Anzahl K an dreidimensionalen Bildpunkten umfasst. Im Folgenden werden die Begriffe "Satz" und "Vektor" gleichbedeutend verwendet. Symbolzeichen für Vektoren und Matrizen werden durch Fettdruck kenntlich gemacht. Die Eingangsbildpunkte in den Bilddaten der ersten Form können dabei manuell oder automatisiert oder auch in einer geeigneten semi-automatischen Weise bestimmt werden.

In einem zweiten Schritt des erfindungsgemäßen Verfahrens erfolgt eine prognostizierende Bestimmung eines Satz an Zielformparametern eines Zielformmodells mit einer definierten Mehrzahl L an Zielformparametern durch ein datengetriebenes Regressionsverfahren aus der Mehrzahl K an Eingangsbildpunkten. Dabei ist in vorteilhafter Weise die Mehrzahl L an Zielformparametern kleiner als die Mehrzahl K an Eingangsbildpunkten. Als Zielformmodell wird hier ein mathematisches Modell bezeichnet, welches Bilddaten der zweiten Form in einer möglichst kompakten Weise beschreiben kann, um damit den Aufwand bei der Bestimmung der zweiten Form an Bilddaten gering zu halten, so dass die Ausführung des erfindungsgemäßen Verfahrens in besonders zeit- und damit kosteneffizienter Weise möglich ist. Dies wird dadurch erreicht, dass das Zielformmodell die Eigenschaften der Bilddaten mit einer Mehrzahl L an Zielformparametern wiedergibt, wobei der Wert für L so gewählt wird, dass die Zielformparameter die Bilddaten der zweiten Form hinreichend genau repräsentieren, aber der Wert für L zugleich geringer ist als die Mehrzahl K an Bildpunkten der Eingangsbildpunkte und auch geringer ist als eine Mehrzahl M an Bildpunkten, welche die Bilddaten der zweiten Form bestimmen. Insbesondere kann ein einzelner Zielformparameter des Zielformmodells auch eine Mehrzahl an Bildpunkten in den Bilddaten der zweiten Form beschreiben, wodurch sich die gewünschte Effizienz bei der Bestimmung der Bilddaten der zweiten Form ergibt.

Die Bestimmung der Mehrzahl L an Zielformparametern aus der Mehrzahl K an Eingangsbildpunkten erfolgt bei dem erfindungsgemäßen Verfahren durch ein Regressionsverfahren. Gegenüber einem rein analytischen Verfahren kann durch die Anwendung eines Regressionsverfahrens in vorteilhafter Weise erreicht werden, dass für alle denkbaren Ausprägungen der betrachteten Körperbereiche des Untersuchungsobjekts ein Satz an Zielformparametern bestimmbar oder zumindest abschätzbar ist. Das Verfahren stellt also durch die Verwendung eines Regressionsverfahrens sicher, dass für alle Untersuchungsobjekte Bilddaten der zweiten Form bestimmt werden können.

Im Besonderen handelt es sich bei dem Regressionsverfahren um ein datengetriebenes Regressionsverfahren, das heißt, dass das Regressionsverfahren aus existierenden radiologischen Bilddaten, sog. Trainingsbilddaten, abgeleitet wurde. Entsprechend werden für das Regressionsverfahren keine Annahmen über die Eigenschaften des Untersuchungsobjekts gemacht. Insbesondere ist es nicht notwendig, zur Festlegung des Regressionsverfahrens vorab biomechanische Parameter, wie beispielsweise die Elastizität von Muskelgewebe, zu bestimmen. Damit ist das erfindungsgemäße Verfahren in vorteilhafter Weise für viele oder alle Körperbereiche des Untersuchungsobjekts einsetzbar.

Die der Ableitung des Regressionsverfahrens zugrunde liegenden Trainingsbilddaten umfassen typischerweise Bilddaten von Untersuchungsobjekten, die jeweils paarweise in der ersten und in der zweiten Form vorliegen. Das Regressionsverfahren wird mit Hilfe der Trainingsbilddaten bestimmt beziehungsweise parametrisiert und dient in der Folge der prognostizierenden Bestimmung von Bilddaten der zweiten Form für Bilddaten, die nur in der ersten Form vorliegen.

In einem dritten Schritt des erfindungsgemäßen Verfahrens wird die zweite Form von Bilddaten aus dem Satz an Zielformparametern bestimmt. Die im zweiten Schritt des Verfahrens bestimmten Zielformparameter legen die Eigenschaften des Zielformmodells fest, so dass aus dem nun parametrisierten Zielformmodell in einfacher Weise Bilddaten ableitbar sind. Beispielsweise können aus einem parametrisierten dreidimensionalen Zielformmodell durch Schnittbildung mit geometrischen Ebenen zweidimensionale Bilddaten der zweiten Form einfach erzeugt werden. Diese zweidimensionalen Bilddaten der zweiten Form können dann z.B. einem Chirurgen bei der Durchführung operativer Eingriffe wertvolle Hilfe bei der Identifikation kritischer Gewebestrukturen geben.

Das erfindungsgemäße Verfahren lässt sich bei einer Vielzahl an Ausprägungen radiologischer Bilddaten beziehungsweise einer Vielzahl an Anwendungsfällen in der radiologischen Bildgebung vorteilhaft einsetzen. Beispielsweise könnten die Bilddaten der ersten Form bei einer Bilddatenaufnahme entstanden sein, bei der sich der Patient in Bauchlage, d.h. auf dem Bauch liegend, befand. Ein folgender medizinischer Eingriff erfolgt aber meist an Patienten in Rückenlage. Mit Hilfe des erfindungsgemäßen Verfahrens lassen sich nun besonders vorteilhaft die Bilddaten der ersten Form in Bauchlage in Bilddaten der zweiten Form in Rückenlage umwandeln, ohne dass dazu weitere Bilddatenaufnahmen des Patienten in Rückenlage notwendig wären. Dadurch werden die Zeit- und Kostenaufwände in der radiologischen Bildgebung verringert und die radiologische Belastung der Patienten, z.B. durch Röntgenstrahlen, reduziert. Weiterhin kann das erfindungsgemäße Verfahren angewandt werden, um Bilddaten, die von Patienten in einer stehenden Position erzeugt wurden, in Bilddaten umzuwandeln, die einer liegenden Position entsprechen. Darüber hinaus ist es möglich, dreidimensionale Bilddaten eines Patienten in der ersten Form, welche durch ein Röntgen-Tomosynthese-Verfahren erzeugt wurden, in Bilddaten der zweiten Form umzuwandeln, die in einigen Fällen anstelle von Bilddaten aus dreidimensionalen MR-Verfahren genutzt werden können, so dass auf eine zusätzliche zeit- und kostenintensive MR-Bildgebung an diesem Patienten verzichtet werden kann.

Eine erfindungsgemäße Bildbearbeitungsstation in der radiologischen Bildgebung zur Bestimmung einer zweiten Form von Bilddaten aus einer ersten Form von Bilddaten eines Untersuchungsobjekts umfasst eine Eingangsbildpunkteinrichtung zur Bestimmung eines Satzes einer definierten Mehrzahl K an Eingangsbildpunkten in den Bilddaten der ersten Form. Weiterhin umfasst die erfindungsgemäße Bildbearbeitungsstation eine Prognoseeinrichtung zur Bestimmung eines Satzes an Zielformparametern eines Zielformmodells mit einer definierten Mehrzahl L an Zielformparametern durch ein datengetriebenes Regressionsverfahren aus der Mehrzahl K an Eingangsbildpunkten, wobei die Mehrzahl L an Zielformparametern kleiner ist als die Mehrzahl K an Eingangsbildpunkten. Darüber hinaus umfasst die erfindungsgemäße Bildbearbeitungsstation eine Bilddatenbestimmungseinrichtung zur Bestimmung der zweiten Form von Bilddaten aus dem Satz an Zielformparametern. Die Eingangsbildpunkteinrichtung, die Prognoseeinrichtung oder die Bilddatenbestimmungseinrichtung können dabei teilweise oder vollständig durch Hardware-Komponenten ausgeführt sein, beispielsweise unter Verwendung von Halbleiter-Bausteinen wie ASICs (Application Specific Integrated Circuits), FPGAs (Field Programmable Gate Arrays), oder PLAs (Programmable Logic Arrays).

Die abhängigen Ansprüche sowie die nachfolgende Beschreibung enthalten besonders vorteilhafte Weiterbildungen und Ausgestaltungen der Erfindung, wobei insbesondere auch die Ansprüche einer Kategorie analog zu den abhängigen Ansprüchen einer anderen Anspruchskategorie weitergebildet sein können.

Bevorzugt ist das erfindungsgemäße Verfahren so ausgebildet, dass der Satz an Zielformparametern eine Abweichung von einem standardisierten geometrischen Zielformmodell beschreibt. Ein für eine bestimmte Bilddatenaufnahme spezifisches Zielformmodell kann damit als Summe aus einem standardisierten geometrischen Zielformmodell und den Abweichungen von diesem standardisierten Zielformmodell beschrieben werden. Wird beispielsweise das Zielformmodell durch einen Satz oder Vektor s beschrieben, welcher eine Mehrzahl N an Bildpunkten enthält und wird das standardisierte geometrische Zielformmodell durch einen Satz oder Vektor $\mathbf{s_M}$ beschrieben, welcher ebenfalls eine Mehrzahl an Bildpunkten N enthält, dann kann der Vektor $s$ des Zielformmodells zumindest näherungsweise wie folgt bestimmt werden:

$$\mathbf{s} = \mathbf{s_M} + \mathbf{P} \cdot \mathbf{y} .$$

Hierbei ist $\mathbf{y}$ der Satz oder Vektor an Zielformparametern der Länge L und $\mathbf{P}$ eine Matrix, welche sich aus den Basisvektoren des Zielformmodellraums zusammensetzt. Für den Fall, dass die Bilddaten in dreidimensionaler Ausprägung vorliegen, enthalten der Vektor $\mathbf{s}$ und der Vektor $\mathbf{s_M}$ jeweils N Bildpunkte, wobei jeder Bildpunkt $i \in \{1, 2, \dots, N\}$ durch drei Zahlenwerte $(x_i, y_i, z_i)$ dargestellt wird, so dass in diesem Fall für den Vektor $\mathbf{s}$ und $\mathbf{s_M}$ gilt:

$$\mathbf{s} \;=\; (x_1,\; y_1,\; z_1,\; x_2,\; y_2,\; z_2,\; \ldots,\; x_N,\; y_N,\; z_N)^T$$

und

$$\mathbf{s_M} \;=\; (x_{1M},\; y_{1M},\; z_{1M},\; x_{2M},\; y_{2M},\; z_{2M},\; \ldots,\; x_{NM},\; y_{NM},\; z_{NM})^T \;.$$

Dabei handelt es sich bei den Zahlenwerten $x_i$, $y_i$ und $z_i$ nicht notwendigerweise um Koordinaten in einem kartesischen Raum. Andere Raumdarstellungen, wie beispielsweise Zylinder- oder Kugelkoordinatensysteme sind im Rahmen des erfindungsgemäßen Verfahrens ebenso anwendbar. Die Matrix **P** aus Basisvektoren kann unter anderem aus existierenden radiologischen Bilddatensammlungen oder sog. Trainingsbilddatensätzen bestimmt werden, welche die Bilddaten der zweiten Form eines Untersuchungsobjekts enthalten. Da dann das standardisierte geometrische Zielformmodell auf existierenden Trainingsbilddatensätzen basiert, lässt es sich auch als sog. statistisches Zielformmodell bezeichnen. Zur Bestimmung der Matrix **P** kann bevorzugt das Verfahren der Hauptkomponentenanalyse (principal component analysis, PCA) vorteilhaft eingesetzt werden.

Besonders bevorzugt wird das standardisierte geometrische Zielformmodell durch Mittelwertbildung aus den gegebenen radiologischen Trainingsbilddatensätzen bestimmt. Für eine gegebene Anzahl J an Trainingsbilddaten $\mathbf{s_t}$ mit $t \in \{1, 2, ..., J\}$ mit:

$$\mathbf{s_t} \;=\; (x_{1t},\; y_{1t},\; z_{1t},\; x_{2t},\; y_{2t},\; z_{2t},\; \ldots,\; x_{Nt},\; y_{Nt},\; z_{Nt})^T$$

ergibt sich damit der Vektor $\mathbf{s_M}$ des standardisierten geometrischen Zielformmodells als:

$$\mathbf{s_M} \;=\; (\mathbf{s_1} + \mathbf{s_2} + \mathbf{s_3} + \ldots + \mathbf{s_J}) \;/\; J \;.$$

Neben dieser sog. arithmetischen Mittelwertbildung sind für das erfindungsgemäße Verfahren auch andere Mittelungsverfahren denkbar.

Bevor der Mittelwert berechnet wird, werden bevorzugt alle Trainingsbilddatensätzen einigermaßen aliniert, um eine geringere Varianz im statistischen Zielformmodell zu erhalten. Hierbei kann bevorzugt die Methode der sog. Generalisierten Procrustes Analyse (GPA) angewandt werden.

In einer bevorzugten Ausführungsform ist das erfindungsgemäße Verfahren dadurch gekennzeichnet, dass die Bestimmung eines Satzes einer definierten Mehrzahl K an Eingangsbildpunkten in den Bilddaten der ersten Form zumindest einen der folgenden drei Verfahrensschritte umfasst. Zunächst wird die erste Form der Bilddaten durch einen Segmentierungsverfahrensschritt bearbeitet. Das heißt im Wesentlichen, dass einzelne Bereiche in den Bilddaten zu Segmenten zusammengefasst werden, bzw. in Segmenten gruppiert werden. Für Bilddaten die in zweidimensionaler Form vorliegen heißt das, dass benachbarte Bilddaten-Pixel in einem Segment zusammengefasst werden, sofern sie bestimmte Bedingungen, welche auch als Homogenitätskriterien bezeichnet werden, erfüllen. So könnten Pixel, deren Grauwerte ähnlich sind, d.h. deren Grauwerte beispielsweise auf eine ähnliche Schwächung von Röntgenstrahlen beim Durchlaufen eines Untersuchungsobjekts hinweisen, in einem gemeinsamen Segment zusammengefasst werden. Durch eine derartige Segmentierung lassen sich in den Bilddaten somit oft Organe, zusammenhängende Knochenstrukturen oder beispielsweise die Hautoberfläche des Untersuchungsobjekts in einem Segment zusammenfassen. Eine den Pixeln entsprechende Zusammenfassung kann für die sog. Voxel angewandt werden, um dreidimensionalen Bilddaten zu segmentieren. Besonders bevorzugt wird die Segmentierung dabei automatisch, das heißt ohne manuelle Eingriffe eines Bedieners, oder zumindest weitestgehend automatisch mit einer eher geringen Anzahl an manuellen Eingriffen erfolgen. Für eine derartige Ausführung der Segmentierung eignen sich bevorzugt Schwellwertbildungsverfahren ("Thresholding") oder Regionenwachstumsverfahren ("Region growing"). Bei Letzterem geht das Verfahren von einem oder mehreren Start- oder Saatpunkten aus, d.h. im Fall von zwei- oder dreidimensionalen Bilddaten von Saat-Pixeln oder Saat-Voxeln. Anschließend werden an die Saat-Pixel bzw. Saat-Voxel angrenzende Pixel oder Voxel immer dann zu der Menge an Saat-Pixeln oder Saat-Voxeln hinzugefügt, wenn sie ein Homogenitätskriterium erfüllen.

Neben dem Segmentierungsverfahrensschritt wird in der bevorzugten Ausführungsform der Bestimmung eines Satzes an Eingangsbildpunkten eine Anzahl an anatomischen Landmarken in der ersten Form der Bilddaten bestimmt. Besonders bevorzugt erfolgt eine derartige Bestimmung mit automatischen oder teilweise automatischen Detektionsverfahren des maschinellen Lernens, wobei die zu detektierenden Landmarken innerhalb der Bilddaten besonders bevorzugt als 3-D Haar-Merkmale, basierend auf den Bilddaten der ersten Form, repräsentiert werden.

Weiterhin ist die bevorzugte Ausführungsform der Bestimmung eines Satzes Eingangsbildpunkten durch die Bestimmung einer Anzahl an Grenzflächen in den segmentierten Bilddaten der ersten Form, bevorzugt nach einem Marching-Cube-Verfahren, gekennzeichnet, wobei die geometrische Lage der Grenzflächen durch die vorab bestimmten anatomischen Landmarken festgelegt wird. Anschließend werden die Eingangsbildpunkte in der ersten Form der Bilddaten so bestimmt, dass zumindest ein Teil der Eingangsbildpunkte auf den vorab bestimmten Grenzflächen liegt.

Bevorzugt ist das erfindungsgemäße Verfahren dadurch gekennzeichnet, dass der Satz an Eingangsbildpunkten so bestimmt wird, dass die Eingangsbildpunkte näherungsweise gleichmäßig auf einer Anzahl an Grenzflächen des Körperbereichs verteilt sind. Insbesondere können die Eingangsbildpunkte in einer Mehrzahl an Gruppen angeordnet sein, wobei die Eingangsbildpunkte innerhalb einer Gruppe sich in gleichem räumlichem Abstand von einer anatomischen Landmarke befinden. Eine Gruppe kann dabei auch nur aus einem Eingangsbildpunkt bestehen.

In einer bevorzugten Ausführungsform des erfindungsgemäßen Verfahrens ist das Verfahren so ausgebildet, dass es vorteilhaft auf Körperbereiche des Untersuchungsobjekts anwendbar ist, die als Paar an dem Untersuchungsobjekt auftreten. Dies betrifft sowohl äußere Organpaare des Untersuchungsobjekts, wie Augen, Ohren, Hände, Arme, Beine, Füße, Brüste, usw. als auch innere Organpaare des Untersuchungsobjekts, wie Lungenflügel, Nieren, Herzkammern, Gehirnhälften, usw. Dazu wird zur Anwendung des Verfahrens für den zweiten Teil des Paares das Verfahren für den ersten Teil angewandt, wobei hier vor der Bestimmung des Satzes an Eingangsbildpunkten die Bilddaten der ersten Form des zweiten Teils des Paares an einer Spiegelachse gespiegelt werden und nach der Bestimmung der zweiten Form der Bilddaten diese erneut an einer Spiegelachse gespiegelt werden. Das heißt, dass sich durch diese Ausführungsform ein erfindungsgemäßes Verfahren, welches für den erste Teil eines Paares eines Körperbereiches erstellt wurde, mit geringem Aufwand auf den zweiten Teil des Paares anwendbar ist, ohne dass dazu das Verfahren für den zweiten Teil des Paares entwickelt werden muss. Insbesondere ist es vorteilhafterweise nicht notwendig, dass für den zweiten Teil des Paares des Körperbereichs des Untersuchungsobjekts Trainingsbilddatensätze vorliegen.

Bevorzugt ist das erfindungsgemäße Verfahren einsetzbar, wenn Körperbereiche des Untersuchungsobjekts während der Aufnahme der ersten Form der Bilddaten durch einen äußeren Mechanismus einer Verformung ausgesetzt sind und die zweite Form der Bilddaten die Körperbereiche ohne diese Verformung darstellen soll. Derartige äußere Mechanismen zur Verformung oder Kompressionen eines Körperbereichs werden u.a. eingesetzt, um den Körperbereich in einer bestimmten Position während der Bilddatenaufnahme zu fixieren oder um das bei der Bildgebung durchstrahlte Körpervolumen zu reduzieren, bzw. um eine definierte Durchstrahlungsdicke bei der Bildgebung zu gewährleisten. Mit Hilfe des erfindungsgemäßen Verfahrens können damit Bilddaten eines Körperbereichs in nicht komprimierter Darstellung aus dessen Bilddaten in komprimierter Darstellung erzeugt werden.

Besonders bevorzugt ist das erfindungsgemäße Verfahren für die Anwendung an einer weiblichen Brust vorgesehen, das heißt, dass der der Verformung ausgesetzte Körperbereich eine Brust umfasst. Bei den Bilddaten der ersten Form handelt es sich dann vorzugsweise um Bilddaten, welche durch ein Verfahren der Brusttomosynthese ermittelt wurden. Bei einer Brusttomosynthese, insbesondere der digitalen Brusttomosynthese (DBT), werden von einer Patientin häufig zwei oder mehr Aufnahmen in unterschiedlichen Positionen erstellt. Die Brust wird dabei während der Bilddatenaufnahme durch einen Mechanismus, der plattenähnliche Strukturen umfasst, beispielsweise durch ein sog. Kompressions-Paddle, komprimiert. In der medizinischen Praxis gängige Aufnahmepositionen sind die sog. "Mediolateral-Oblique" (MLO)-Position und die sog. "Cranio-Caudal"(CC)-Position. Die Aufnahmepositionen unterscheiden sich durch den geometrischen Winkel, unter dem die Bilddaten erzeugt werden, wobei hierzu insbesondere Röntgeneinrichtungen verwendet werden, bei denen die Röntgenröhre schwenkbar ist, beispielsweise in Winkelschritten von +/- 25°. Mit Hilfe von geeigneten Rekonstruktionsverfahren lassen sich dann aus zweidimensionalen Bilddaten mit unterschiedlichen Aufnahmewinkeln dreidimensionale Bilddaten erzeugen und anschließend mit Hilfe des erfindungsgemäßen Verfahrens in dreidimensionale Bilddaten in unkomprimierter Darstellung umwandeln. Die Bilddaten der zweiten Form sind dann vorzugsweise Bilddaten der Brust in unkomprimierter Form, z.B. einer stehenden oder liegenden Patientin, oder in einer auf dem Bauch liegenden Position, beispielsweise in einem MR-Gerät. Grundsätzlich können aber auch die Bilddaten der zweiten Form die Brust in einer durch äußere Einflüsse, z.B. durch eine Brustspule im MR-Gerät und/oder Gravitation, verformten Lage zeigen.

Das heißt, dass es durch das erfindungsgemäße Verfahren möglich wird, Bilddaten von relativ kostengünstigen Mammographie-Einrichtungen für operative Eingriffe, welche an der Brust in unkomprimierter Form stattfinden, zu verwenden, ohne dass die Bilddaten durch die Kompression verfälscht sind. Neben der genannten Kompression der Brust während einer Mammographie können die Bilddaten der ersten Form auch von einer Bilddatenaufnahme stammen, bei der die Patientin sich in Bauchlage befand und die Brust durch die Gravitation und/oder Brustspule verformt ist. Dies gilt insbesondere für die sog. "Face-Down"-MR-Tomographie-Bildgebung. Auch in diesem Fall lassen sich mit Hilfe des erfindungsgemäßen Verfahrens Bilddaten der zweiten Form bestimmen, die einer nicht verformten Position, bzw. einer Bilddatenaufnahme in Rückenlage entsprechen.

Bevorzugt wird der Satz an Eingangsbildpunkten durch das erfindungsgemäße Verfahren so bestimmt, dass die Eingangsbildpunkte auf der Oberfläche der Brust liegen und besonders bevorzugt die Eingangsbildpunkte Gruppen (mit einem oder mehreren Eingangsbildpunkten) zugeordnet sind, wobei die Eingangsbildpunkte innerhalb einer Gruppe in etwa den gleichen Abstand von der Papilla der Brust aufweisen. Dabei kann das Verfahren Schritte zur zumindest teilweise automatischen Bestimmung der Papilla der Brust umfassen.

Die prognostizierende Bestimmung der Mehrzahl L an Zielformparametern aus der Mehrzahl K an Eingangsbildpunkten erfolgt bei dem erfindungsgemäßen Verfahren durch ein Regressionsverfahren, d.h. es wird durch das Regressionsverfahren aus dem Vektor x mit K Eingangsbildpunkten ein Vektor y mit einer Mehrzahl L an Zielformparametern bestimmt. Da die Zahl der Eingangsbildpunkte größer als Eins ist, können bevorzugt sog. multiple Regressionsverfahren vorteilhaft eingesetzt werden, da diese mehr als eine Eingangsvariable bzw. eine mehrdimensionale Eingangsvariable zur Bestimmung einer Ausgangsvariablen verarbeiten können. Weiterhin lassen sich bevorzugt sog. multivariate Regressionsverfahren vorteilhaft einsetzen, da diese Regressionsverfahren dadurch gekennzeichnet sind, dass sie mehr als eine Ausgangsvariable bzw. eine mehrdimensionale Ausgangsvariable bestimmen können. Ein multiples, multi-variates Regressionsverfahren stellt also eine Funktion f bereit, für die gilt:

$$f: \chi \to Y.$$

Dabei ist:

$$f(\mathbf{x}) = \mathbf{y} \text{ mit } \mathbf{x} \in \chi \text{ und } \mathbf{y} \in Y.$$

Dabei repräsentiert $\chi$ den K-dimensionalen reellwertigen Vektorraum und $Y$ den L-dimensionalen reellwertigen Vektorraum.

Besonders bevorzugt ist das Regressionsverfahren dadurch gekennzeichnet, dass es als multiple, multi-variate Random-Forest-Regression ausgebildet ist. Wird diese Regression auf einen Vektor **x** mit K Eingangsbildpunkten angewandt, so werden mit den in diesem Regressionsverfahren hinterlegten Regressionsbäumen - ausgehend von den Wurzeln der Regressionsbäume - Endpunkte oder Blätter der Regressionsbäume bestimmt, welche den Vektor **y** mit seinen L Zielformparametern festlegen. Vorab festgelegte Schwellwerte oder Entscheidungskriterien definieren dabei die Traversierung der Regressionsbäume von den Wurzeln der Regressionsbäume zu den Blättern der Regressionsbäume während der Verfahrensausführung.

Besonders bevorzugt ist das datengetriebene Regressionsverfahren des erfindungsgemäßen Verfahrens dadurch gekennzeichnet, dass das Regressionsverfahren automatisch mit Verfahren des maschinellen Lernens aus radiologischen Trainingsbilddatensätzen abgeleitet ist.

Eine weitere bevorzugte Ausführungsform des erfindungsgemäßen Verfahrens ist dadurch gekennzeichnet, dass die Mehrzahl L an Zielformparametern wesentlich kleiner ist als die Mehrzahl K an Eingangsbildpunkten. Dadurch lässt sich der Aufwand bei der Bestimmung der Parameter des Zielformparameters in vorteilhafter Weise deutlich verringern, so dass das erfindungsgemäße Verfahren effizient ausführbar ist und entsprechend die Bilddaten der zweiten Form ohne längere Wartezeiten verfügbar sind. Um jedoch trotz der relativ geringen Anzahl an Zielformparametern eine gute Qualität der bestimmten Bilddaten der zweiten Form zu erzielen, werden die Zielformparameter bevorzugt so gewählt, dass die kumulative Varianz des Zielformmodells eine kumulative Varianz radiologischer Trainingsbilddatensätze größtenteils, bevorzugt mit wenigstens 80% abbildet. Dies kann beispielsweise dadurch erreicht werden, dass die Zielformparameter so bestimmt werden, dass sie die wesentlichen Abweichungen oder Abweichungsrichtungen von einem standardisierten geometrischen Zielformmodell wiedergeben, während die unwesentlichen Abweichungen von dem standardisierten geometrischen Zielformmodell nicht durch die Zielformparameter wiedergegeben werden.

Das erfindungsgemäße Verfahren kann bevorzugt im Rahmen eines Lagebestimmungs-Verfahrens der radiologischen Bildgebung verwendet werden, bei dem die geometrischen Lage einer Anzahl von Zielobjekten in einer zweiten Form von Bilddaten aus der geometrischen Lage der Zielobjekte in einer ersten Form von Bilddaten eines Körperbereichs eines Untersuchungsobjekts bestimmt wird. Mit "Anzahl" wird dabei hier und im Weiteren eine positive natürliche Zahl größer als Null bezeichnet. Ein Zielobjekt steht dabei allgemein für eine Region im betrachteten Körperbereich des Untersuchungsobjekts, welche von besonderem Interesse für die jeweilige medizinische Untersuchung ist. Hierbei kann es sich sowohl um Zielobjekte handeln, wie sie bei gesunden Patienten auftreten, als auch um Zielobjekte, die auf eine Erkrankung hindeuten. Beispielsweise können die Zielobjekte Läsionen, Gewebeveränderungen, Knochenveränderungen oder -brüche, innere Blutungen, gut- und bösartige Geschwüre oder Kalkablagerungen umfassen.

Ein erster Schritt dieses Lagebestimmungs-Verfahrens umfasst dabei die Bestimmung der zweiten Form von Bilddaten aus der ersten Form von Bilddaten des Untersuchungsobjekts, bevorzugt mit Verfahrensschritten nach dem erfindungsgemäßen Verfahren. Alternativ können aber auch andere Verfahren für diesen Schritt genutzt werden. In einem weiteren Schritt wird eine Anzahl an Grenzflächen des Körperbereichs in der ersten Form und in der zweiten Form der Bilddaten bestimmt. Derartige Grenzflächen können beliebige anatomische Grenzflächen innerhalb des Untersuchungsobjekts sein oder an der äußeren Begrenzung des Untersuchungsobjekts liegen. Dazu zählen beispielsweise die Hautoberfläche, die Schädeldecke, das Zwerchfell, die Darmwand oder die Magenwand. Ein weiterer Schritt des Lagebestimmungs-Verfahrens umfasst die Bestimmung einer Mehrzahl anatomischer Landmarken in den Bilddaten der ersten Form und der zweiten Form. Anatomische Landmarken sind dabei im Allgemeinen anatomische Gegebenheiten des Untersuchungsobjekts die besondere Eigenschaften aufweisen oder die leicht identifizierbar sind. Beispiele für anatomische Landmarken sind Augenwinkel, die Nasenspitze, die Brustwarze (Papilla), ein bestimmter Wirbel der Wirbelsäule, oder die vordere Kommissur (anterior commissure, AC) und hintere Kommissur (posterior commissure, PC) des Gehirns.

In einem weiteren Schritt des Lagebestimmungs-Verfahrens wird eine geometrische Kurve so bestimmt, dass die Kurve zumindest näherungsweise in der Grenzfläche und durch die Mehrzahl anatomischer Landmarken verläuft. Diese Bestimmung findet dabei sowohl in den Bilddaten der ersten Form als auch in den Bilddaten der zweiten Form statt. Anschließend werden Kurvenpunkte in den geometrischen Kurven bestimmt, wobei bevorzugt die Kurvenpunkte zwischen Paaren anatomischer Landmarken gleichmäßig verteilt, also beispielsweise äquidistant angeordnet sind.

In einem weiteren Schritt des Lagebestimmungs-Verfahrens wird eine Mehrzahl an Konturen in den Grenzflächen der ersten und zweiten Form der Bilddaten bestimmt, wobei die Bestimmung der Konturen so gewählt wird, dass jeweils eine Kontur durch einen Kurvenpunkt verläuft. Bevorzugt handelt es sich bei den Konturen um sog. Splines. Im mathematischen Sinn ist ein Spline eine Kurve, die durch eine bestimmte Anzahl von Punkten verläuft und diese "glatt" miteinander verbindet. Splines sind im Allgemeinen dem Fachexperten im Bereich des Maschinenbaus bekannt. Sie werden dort zur Beschreibung von geometrischen Formen, beispielsweise von Schiffsrümpfen oder Karosserieteilen der Automobiltechnik eingesetzt.

Die geometrische Lage der genannten Zielobjekte in der zweiten Form der Bilddaten wird in einem weiteren Schritt des Lagebestimmungs-Verfahrens in Abhängigkeit von den Konturen in der zweiten Form der Bilddaten durch eine Interpolation, bevorzugt durch Thin-Plate-Spline-Interpolationen (TPS-Interpolation), zwischen den Konturpunkten der ersten Form der Bilddaten und der zweiten Form der Bilddaten beschrieben. Erfindungsgemäß werden die Thin-Plate-Splines dabei vorteilhaft verwendet, um basierend auf den oben erwähnten anatomische Grenzflächen die Deformation des Volumens beim Umwandeln der Bilddaten von der einen in die andere Form zu beschreiben.

Das Lagebestimmungs-Verfahren ermöglicht es in vorteilhafter Weise medizinisch relevante Zielobjekte so in Bilddaten darzustellen, dass diese bei medizinischen Eingriffen, beispielsweise präventiven oder therapeutischen Gewebeentnahmen, einfach und mit guter geometrischer Genauigkeit für das medizinische Fachpersonal in Bilddaten identifizierbar und lokalisierbar sind. Dies gilt insbesondere dann, wenn die Bilddaten der ersten Form in einer anderen Position des Untersuchungsobjekts oder des betreffenden Körperbereiches aufgenommen wurden als die Position, in der die Gewebeentnahme stattfindet. Werden beispielsweise bei einer Bilddatenaufnahme in einer stehenden Position des Untersuchungsobjekts kritische Zielobjekte durch das medizinische Fachpersonal identifiziert, so können diese Zielobjekte mit dem erfindungsgemäßen Verfahren in Bilddaten einer zweiten Form dargestellt werden, welche einer anders gearteten Position des Untersuchungsobjekts entsprechen.

Zur Durchführung des Lagebestimmungs-Verfahrens kann eine Zielobjektbestimmungseinrichtung genutzt werden, welche eine Schnittstellenanordnung zur Übernahme von Bilddaten des Untersuchungsobjekts in einer ersten Form sowie von Daten betreffend Zielobjekten in der ersten Form der Bilddaten und zur Übernahme von Bilddaten in einer zweiten Form aus einer erfindungsgemäßen Bildbearbeitungsstation aufweist. Diese Zielobjektbestimmungseinrichtung muss dann ausgebildet sein, um folgende Schritte durchzuführen:

- Bestimmung einer Anzahl an Grenzflächen des Körperbereichs in der ersten Form der Bilddaten und in der zweiten Form der Bilddaten,
- Bestimmung einer Mehrzahl anatomischer Landmarken in den Bilddaten der ersten Form und in den Bilddaten der zweiten Form,
- Bestimmung einer geometrischen Kurve in der ersten Form der Bilddaten und in der zweiten Form der Bilddaten, wobei die Kurven jeweils zumindest näherungsweise in der Grenzfläche und durch die Mehrzahl anatomischer Landmarken verlaufen,
- Bestimmung von Kurvenpunkten in den geometrischen Kurven,
- Bestimmung einer Mehrzahl an Konturen in der Grenzfläche der ersten Form der Bilddaten und in der Grenzfläche der zweiten Form der Bilddaten,
- Beschreibung der geometrischen Lage der Zielobjekte in den Bilddaten der ersten Form in Abhängigkeit von den Konturen der ersten Form der Bilddaten,
- Bestimmung der geometrischen Lage der Zielobjekte in den Bilddaten der zweiten Form in Abhängigkeit von den Konturen der zweiten Form der Bilddaten, unter Anwendung einer Interpolationen zwischen Konturen der ersten Form der Bilddaten und Konturen der zweiten Form der Bilddaten.

Die Zielobjektbestimmungseinrichtung kann dabei auch derart in die erfindungsgemäße Bildbearbeitungsstation integriert sein, dass sie die in verschiedenen Zwischenschritten des erfindungsgemäßen Verfahrens erzeugten Zwischenergebnisse wie z.B. anatomische Landmarken oder Grenzflächen bei der Zielobjektbestimmung mit benutzt.

Eine erfindungsgemäße Bildgebungseinrichtung, beispielsweise ein Ultraschallsystem, ein Röntgengerät, ein Mammographie-System, ein Röntgen-Computertomographie(CT)-System, ein Positronen-Emissionstomographie(PET)-System, ein Single-Photon-Emissionstomographie(SPECT)-System oder Magnetresonanz(MR)-System ist durch eine erfindungsgemäße Bildbearbeitungsstation und/oder Zielobjektbestimmungseinrichtung gekennzeichnet. Eine technische Umsetzung der erfindungsgemäßen Verfahren kann auf verschiedenste Arten und Weisen erfolgen.

EP 2 634 748 B1

Insbesondere ist es denkbar, dass eine Umsetzung zumindest teilweise mit Hilfe elektrischer Schaltkreise wie ASICs (Application Specific Integrated Circuits), FPGAs (Field Programmable Gate Arrays), oder PLAs (Programmable Logic Arrays) erfolgt. Darüber hinaus kann ein Computerprogrammprodukt, welches direkt in einen Speicher einer programmierbaren Bildgebungseinrichtung und/oder Bildbearbeitungsstation ladbar ist, die erfindungsgemäßen Verfahren zumindest teilweise mit Programmcode-Mitteln ausführen, wenn das Computerprogrammprodukt in der Bildgebungseinrichtung bzw. Bildbearbeitungsstation ausgeführt wird.

Die Erfindung wird im Folgenden unter Hinweis auf die beigefügten Figuren anhand von Ausführungsbeispielen noch einmal näher erläutert. Dabei werden gleichartige Komponenten mit denselben Bezugsziffern bezeichnet. Es zeigen:

Figur 1      eine erfindungsgemäße Bildbearbeitungsstation,

Figur 2      eine Mammographie-Einrichtung,

Figur 3      beispielhafte Bilddaten der ersten und der zweiten Form für das erfindungsgemäße Verfahren,

Figur 4      ein Beispiel für das erfindungsgemäße Verfahren zur Bestimmung der Eingangsbildpunkte,

Figur 5      ein Beispiel eines Regressionsbaums eines beispielhaften Regressionsverfahrens,

Figur 6      ein Beispiel für die Bestimmung einer ersten Zielvariablen des beispielhaften Regressionsverfahrens,

Figur 7      ein Beispiel für die Bestimmung einer zweiten Zielvariablen des beispielhaften Regressionsverfahrens,

Figur 8      ein Beispiel für die kumulative Varianz des Zielformmodells als Funktion der Mehrzahl an Zielformparametern,

Figur 9      ein Anwendungsbeispiel für das erfindungsgemäße Verfahren zur Bestimmung von Kurvenpunkten,

Figur 10     ein Beispiel mit Bilddaten der ersten Form und den zugehörigen Konturenpunkten,

Figur 11     ein Beispiel mit Bilddaten der zweiten Form und den zugehörigen Konturenpunkten,

Figur 12     ein Beispiel mit Bilddaten der zweiten Form und ein durch das erfindungsgemäße Verfahren bestimmtes Zielobjekt.

In Folgenden wird die Erfindung, ohne Beschränkung der Allgemeinheit, bei einer Anwendung an einer weiblichen Brust beschrieben. Dabei umfassen die Bilddaten der ersten Form jeweils Bilddaten aus einer digitalen Brusttomosynthese (DBT), die wie üblich aus Aufnahmen in der "Mediolateral-Oblique" (MLO) -Position und der "Cranio-Caudal" (CC)-Position rekonstruiert worden sind, und die daher auf Bilddaten der entsprechend komprimierten Brust basieren bzw. diese Bilddaten enthalten. Bei den Bilddaten der zweiten Form handelt es sich um Bilddaten der unkomprimierten Brust.

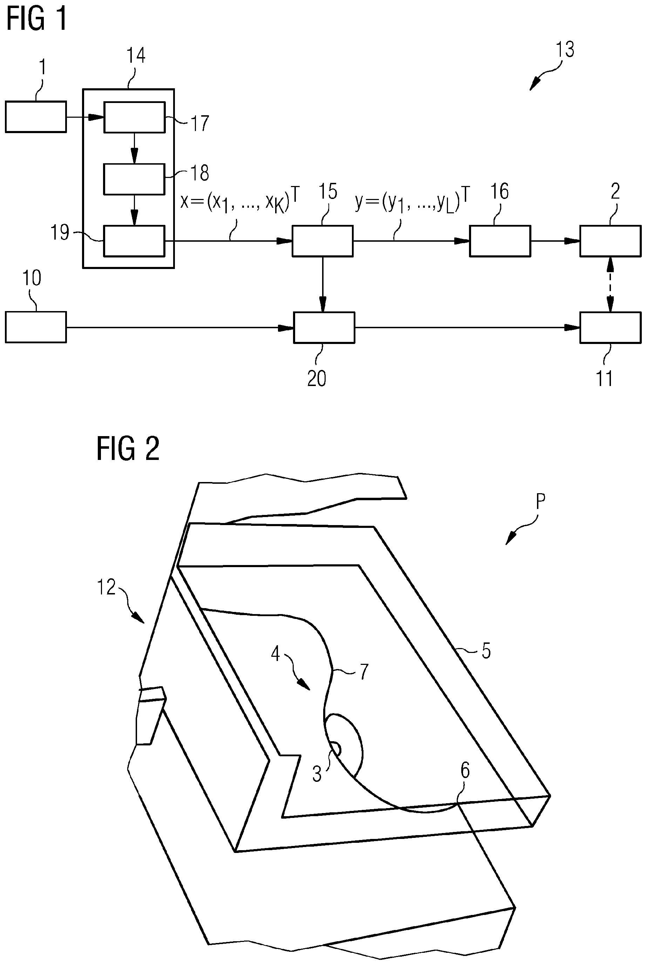

Die Figur 1 zeigt eine erfindungsgemäße Bildbearbeitungsstation 13, welche aus Bilddaten der ersten Form 1 eines Untersuchungsobjektes P Bilddaten der zweiten Form 2 bestimmt. Die Bildbearbeitungsstation 13 weist eine Eingangsbildpunkteinrichtung 14 zur Bestimmung eines Satzes $\mathbf{x}$ einer definierten Mehrzahl K an Eingangsbildpunkten $\mathbf{x}_1, ... , \mathbf{x}_K$ auf. Die Eingangsbildpunkteinrichtung 14 umfasst eine Landmarkenbestimmungseinrichtung 17 zur Bestimmung einer Mehrzahl anatomischer Landmarken in den Bilddaten der ersten Form 1. Dabei kann die Landmarkenbestimmungseinrichtung 17 ebenfalls über geeignete Segmentierungseinrichtungen zur Segmentierung der Bilddaten der ersten Form 1 verfügen. Weiterhin umfasst die Eingangsbildpunkteinrichtung 14 eine Grenzflächenbestimmungseinrichtung 18, die in den, ggf. bereits segmentierten Bilddaten der ersten Form 1, Grenzflächen wie Hautoberfläche, die Schädeldecke, das Zwerchfell, die Darm- oder die Magenwand bestimmt. Darüber hinaus umfasst die Eingangsbildpunkteinrichtung 14 eine Resampling-Einrichtung 19, welche Eingangsbildpunkte $\mathbf{x}_1, ... , \mathbf{x}_K$ bestimmt und diese mit Verfahren des Resamplings in den Bilddaten der ersten Form 1 in einer vorgegebenen Weise verteilt, beispielweise so dass die Eingangsbildpunkte $\mathbf{x}_1, ... , \mathbf{x}_K$ näherungsweise gleichmäßig in einer der Grenzflächen 9 verteilt sind. Die Bildbearbeitungsstation 13 umfasst außerdem eine Prognoseeinrichtung 15, welche einen Satz an Zielformparametern y eines Zielformmodells mit einer definierten Mehrzahl L an Zielformparametern $\mathbf{y}_1, ..., \mathbf{y}_L$ durch ein datengetriebenes Regressionsverfahren aus den Eingangsbildpunkten $\mathbf{x}_1, ... , \mathbf{x}_K$, bestimmt, wobei erfindungsgemäß die Mehrzahl L an Zielformparametern $\mathbf{y}_1, ..., \mathbf{y}_L$ kleiner ist als die Mehrzahl K an Eingangsbildpunkten $\mathbf{x}_1, ... , \mathbf{x}_K$. Aus den so bestimmten Zielformparametern $\mathbf{y}_1, ..., \mathbf{y}_L$ bestimmt eine Bilddatenbestimmungseinrichtung 16 die zweite Form an Bilddaten 2.

Weiterhin kann eine erfindungsgemäße Bildbearbeitungsstation 13 Einrichtungen umfassen, mit denen Zielobjekte 11 in den Bilddaten der zweiten Form 2 bestimmt werden. Fig. 1 zeigt hierzu eine Zielobjektbestimmungseinrichtung 20, welche hier Teil der Bildbearbeitungsstation 13 ist und so ausgebildet ist, dass sie aus Zielobjekten 10 in den Bilddaten der ersten Form 1 Zielobjekte 11 in den Bilddaten der zweiten Form 2 mit den erfindungsgemäßen Verfahren bestimmt. Hierzu übernimmt sie z.B. (wie in Figur 1 symbolisch dargestellt) die erforderlichen Daten, insbesondere die Bilddaten der ersten Form 1 und der zweiten Form 2 von der Prognoseeinrichtung 15 bzw. von den mit der Prognoseeinrichtung 15 verknüpften Einrichtungen.

Die Figur 2 stellt eine Mammographie-Einrichtung 12 dar, wie sie in der medizinischen Praxis zur Erzeugung von Bilddaten einer Brust 4 einer Patientin P üblich ist. Insbesondere werden derartige Mammographie-Einrichtungen 12 verwendet, um DBT-Bilddaten zu generieren. Die Brust 4 wird dabei durch einen mechanischen Mechanismus, das sog. Paddle 5, fixiert und komprimiert. Die Figur 2 zeigt dabei beispielhaft eine Positionierung der Patientin P in der MLO-Position. Weiterhin dargestellt sind die Papilla 3, der Brustansatz 7 und die Unterbrustfalte 6, welche in der medizinischen Praxis auch als Brustumschlagsfalte bezeichnet wird.

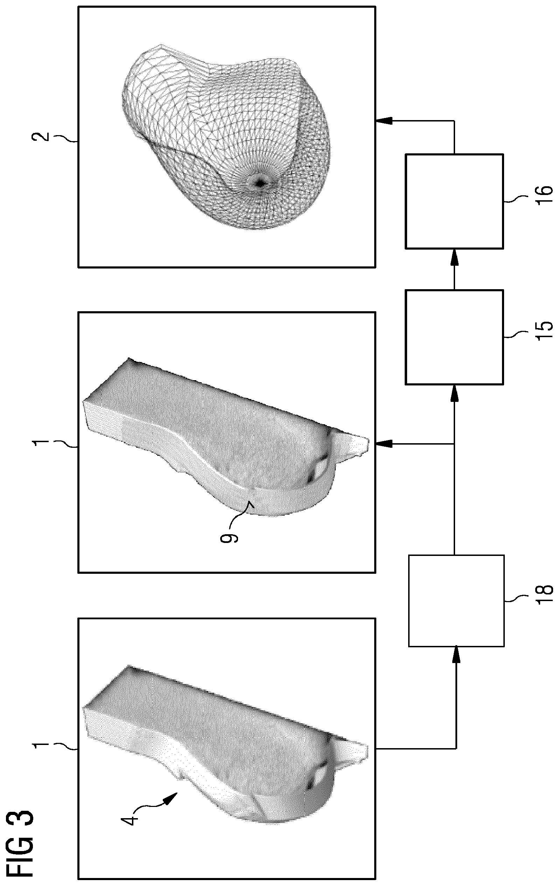

Figur 3 zeigt für das erfindungsgemäße Verfahren beispielhafte Bilddaten der ersten Form 1 und die entsprechenden Bilddaten der zweiten Form 2. Insbesondere wird durch die Figur 3 dargestellt, wie in den Bilddaten der ersten Form 1, welche bereits segmentiert wurden, als Zwischenschritt des Verfahrens mit einer Grenzflächenbestimmungseinrichtung 18 eine Grenzfläche 9 bestimmt wird. Bei der Grenzfläche 9 handelt es sich in diesem Beispiel um die Hautoberfläche einer Brust 4. Weiterhin zeigt die Figur 3 die Bilddaten der zweiten Form 2, welche mit einer Prognoseeinrichtung 15 und einer Bilddatenbestimmungseinrichtung 16 bestimmt wurden. Insbesondere zeigt die Figur 3 dabei, wie aus Bilddaten der ersten Form 1, welche die Brust 4 in komprimierter Form zeigen, ohne weitere Bilddatenaufnahmen in vorteilhafter Weise Bilddaten der zweiten Form 2 in nicht komprimierter Weise durch die Erfindung prognostiziert werden können.

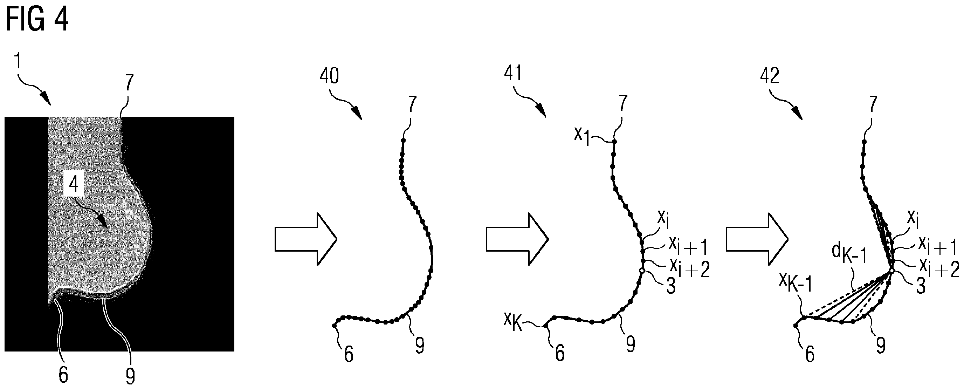

Die Figur 4 zeigt die erfindungsgemäßen Verfahrensschritte zur Bestimmung des Satzes an Eingangsbildpunkten $x_1$, ... , $x_K$ in den Bilddaten der ersten Form 1 am Beispiel von Bilddaten einer Brust 4. Dazu werden in den Bilddaten der ersten Form 1 zunächst charakteristische anatomische Landmarken des betrachteten Körperbereiches der Patientin P bestimmt. In dem für die Figur 4 gewählten Beispiel werden dabei die Unterbrustfalte 6 und der Brustansatz 7 in den Bilddaten der ersten Form 1 identifiziert. Diese Identifikation kann dabei automatisch oder auch mit manueller Unterstützung des medizinischen Fachpersonals erfolgen. Anschließend wird in einem ersten Verfahrensschritt 40 zur Bestimmung der Eingangsbildpunkte $x_1$, ... , $x_K$ eine Grenzfläche 9 extrahiert, welche durch die Unterbrustfalte 6 und den Brustansatz 7 verläuft und welche durch eine Anzahl an Bildpunkten festgelegt ist. In einem weiteren Verfahrensschritt 41 wird die Zahl der Bildpunkte durch Entfernen vorhandener oder Erzeugen zusätzlicher Bildpunkte die Anzahl der Bildpunkte so modifiziert, dass ihre Zahl der definierten Mehrzahl K entspricht. Darüber hinaus werden die Eingangsbildpunkte durch Verfahren des Resampling auf der Grenzfläche 9 positioniert, beispielsweise so, dass sie gleichmäßig auf der Grenzfläche 9 verteilt sind. Insbesondere können in diesem Verfahrensschritt 41 weitere Landmarken bestimmt werden, beispielsweise die Papilla 3. Die gleichmäßige Verteilung der Bildpunkte kann dabei so gewählt werden, dass die Bildpunkte äquidistant zwischen den Landmarken angeordnet sind, beispielsweise also äquidistant zwischen der Papilla 3 und der Unterburstfalte 6, bzw. äquidistant zwischen der Papilla 3 und dem Brustansatz 7. Damit ergeben sich die Eingangsbildpunkte $x_1$, ... , $x_K$. Darüber hinaus werden in einem Verfahrensschritt 42 Abstandsvektoren $d_1$, ... , $d_K$ zwischen einer Anzahl an Landmarken und den Eingangsbildpunkten $x_1$, ... , $x_K$ berechnet. Die Abstandsvektoren $d_1$, ... , $d_K$ eignen sich dabei als Eingangsvariablen für das prognostizierende, datengetriebene Regressionsverfahren zur Bestimmung der definierten Mehrzahl L an Zielformparametern $y_1$, ..., $y_L$.

Bei dem Beispiel in Figur 4 und anderen gezeigten Figuren versteht es sich, dass die erfindungsgemäßen Verfahren und Einrichtungen selbstverständlich auch für dreidimensionale Bilddaten der ersten Form 1 und dreidimensionale Bilddaten der zweiten Form 2 geeignet und vorteilhaft einsetzbar sind, auch wenn hier aus Gründen der Übersichtlichkeit eine zweidimensionale Darstellung gewählt wurde. Insbesondere sind in den Figuren die Grenzflächen 9 und weitere Ebenen aufgrund der hier gewählten Darstellung als Linien gezeigt - ohne jedoch die Anwendung der Erfindung einzuschränken.

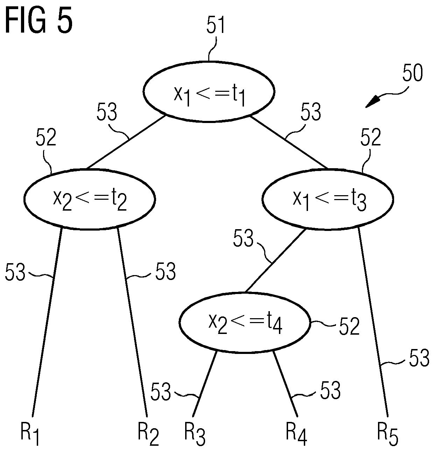

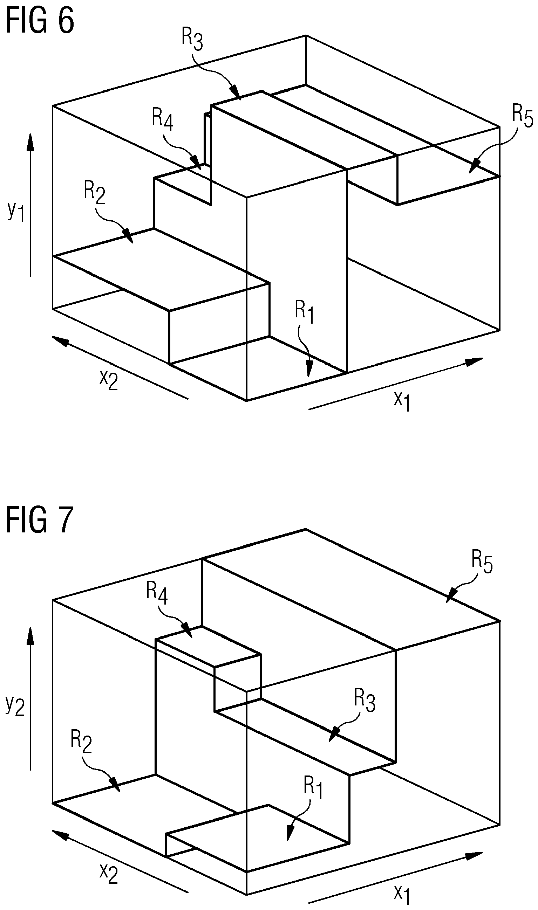

In der Figur 5 wird ein Regressionsbaum 50 eines einfachen, beispielhaften Regressionsverfahrens mit zwei Eingangsvariablen $x_1$ und $x_2$ und zwei Zielvariablen $y_1$ und $y_2$ gezeigt. Dadurch werden die in der Erfindung angewandten Regressionsverfahren, besonders die multiplen, multivariaten Regressionsverfahren näher erläutert. Ein Regressionsbaum 50 umfasst dabei hier eine Wurzel 51, mehrere Knoten 52, mehrere Äste 53 und die Blätter $R_1$, $R_2$, $R_3$, $R_4$, $R_5$. An den Knoten 52 wird bei der Ausführung des Regressionsverfahrens anhand von Schwellwerten $t_1$, $t_2$, $t_3$, $t_4$ entschieden, welche weiteren Äste 53 des Regressionsbaums 50 von der Wurzel 51 hin zu den Blättern $R_1$, $R_2$, $R_3$, $R_4$, $R_5$ traversiert werden. Dabei hängen die Entscheidungen von mehreren Eingangsvariablen $x_1$ und $x_2$ ab, so dass es sich um einen Regressionsbaum 50 für ein multiples Regressionsverfahren handelt. Am Ende des Regressionsverfahrens stehen nach der Traversierung des Regressionsbaums 50 in Abhängigkeit von den Eingangsvariable $x_1$, $x_2$ eine der Regionen $R_1$, $R_2$, $R_3$, $R_4$ oder $R_5$ fest. Anhand dieser Regionen $R_1$, $R_2$, $R_3$, $R_4$, $R_5$ können für die Zielvariablen $y_1$, $y_2$ Werte bestimmt werden, was in der Zusammenschau mit der Figur 6 und der Figur 7 deutlich wird. Da das hier illustrierte Regressionsverfahren mehr als eine Zielvariable $y_1$, $y_2$ aufweist, handelt es sich um ein multi-variates Regressionsverfahren.

In Figur 6 wird ein Beispiel für die Bestimmung einer ersten Zielvariablen $y_1$ des beispielhaften Regressionsverfahrens gezeigt. In Abhängigkeit von den Werten der Eingangsvariablen $x_1$ und $x_2$ wird eine Region $R_1$, $R_2$, $R_3$, $R_4$, $R_5$ bestimmt, welche anschließend den Wert der Zielvariablen $y_1$ festlegt.

Figur 7 zeigt in entsprechender Weise für das Regressionsverfahren die Festlegung des Wertes für die Zielvariable $y_2$.

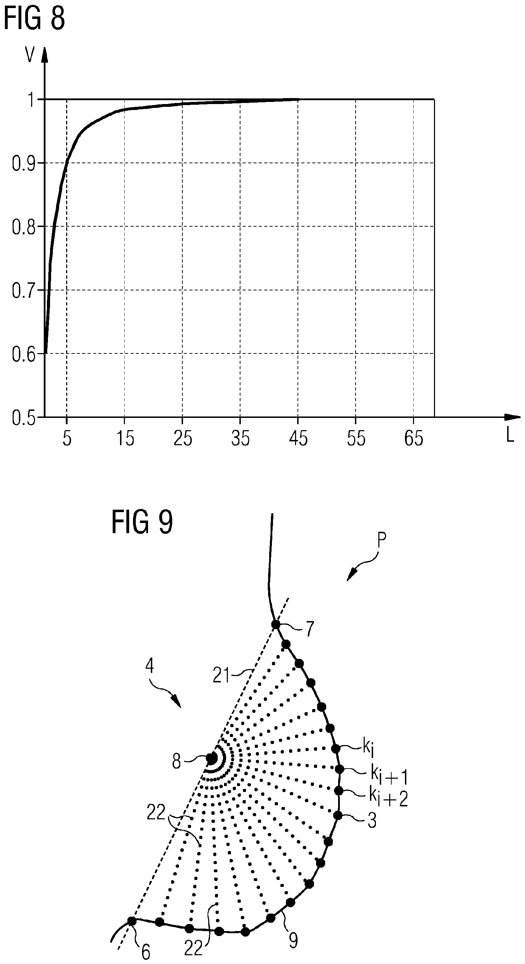

In Figur 8 wird ein Beispiel für die kumulative Varianz V des Zielformmodells als Funktion der Mehrzahl L an Zielformparametern $\mathbf{y}_1$, ..., $\mathbf{y}_L$ gezeigt. Dabei wird deutlich, dass bereits eine relativ geringe Mehrzahl L an Zielformparametern $\mathbf{y}_1$, ..., $\mathbf{y}_L$ ausreicht, um die kumulative Varianz der Bilddaten der zweiten Form 2 abzubilden. Beispielsweise werden gemäß Figur 8 durch nur fünf Zielformparameter $\mathbf{y}_1$, ..., $\mathbf{y}_5$ schon 90% der kumulativen Varianz der Bilddaten der zweiten Form 2 abgebildet. Das heißt, dass das erfindungsgemäße Verfahren mit einer geringen Mehrzahl L an Zielformparametern $\mathbf{y}_1$, ..., $\mathbf{y}_5$ sehr effizient ausgeführt werden kann, ohne dass dadurch wesentliche Einbußen in der Qualität der Prognose von Bilddaten der zweiten Form 2 auftreten.

Die Figur 9 gibt ein Anwendungsbeispiel für das erfindungsgemäße Verfahren zur Bestimmung der Kurvenpunkte $\mathbf{k}_1$, ... , $\mathbf{k}_K$ und der Konturen in den Bilddaten der ersten Form 1 und der zweiten Form 2 einer Brust 4 einer Patientin P wieder. Dazu werden zunächst charakteristische anatomische Landmarken, wie die Papilla 3, die Unterbrustfalte 6 und der Brustansatz 7 in den Bilddaten der ersten Form 1 und der zweiten Form 2 bestimmt. Anschließend erfolgt eine Bestimmung einer Grenzfläche 9, welche durch die bestimmten Landmarken 3, 6, 7 verläuft. Weiterhin wird jeweils eine Kurve k bestimmt, welche durch die Kurvenpunkte $\mathbf{k}_1$, ... , $\mathbf{k}_K$ festgelegt ist, und näherungsweise in der Grenzfläche 9 verläuft sowie durch die anatomischen Landmarken 3, 6, 7 geht. Die Kurvenpunkte $\mathbf{k}_1$, ... , $\mathbf{k}_K$ werden dabei bevorzugt so festgelegt, dass sie auf der Kurve näherungsweise gleichmäßig verteilt sind. Außerdem wird die Brustebene 21 bestimmt, welche senkrecht zur MLO-Ebene steht und welche durch die durch die Unterbrustfalte 6 und den Brustansatz 7 festgelegten Punkte verläuft. Anschließend findet eine Projektion der Papilla 3 auf die Brustebene 21 statt, wodurch sich ein Projektionspunkt 8 der Papilla 3 ergibt. In einem weiteren Schritt werden Ebenen 22, die sich aus Rotation der Brustebene 21 um den Projektionspunkt 8 ergeben, so festgelegt, dass sie durch die Kurvenpunkte $\mathbf{k}_1$, ... , $\mathbf{k}_K$ verlaufen.

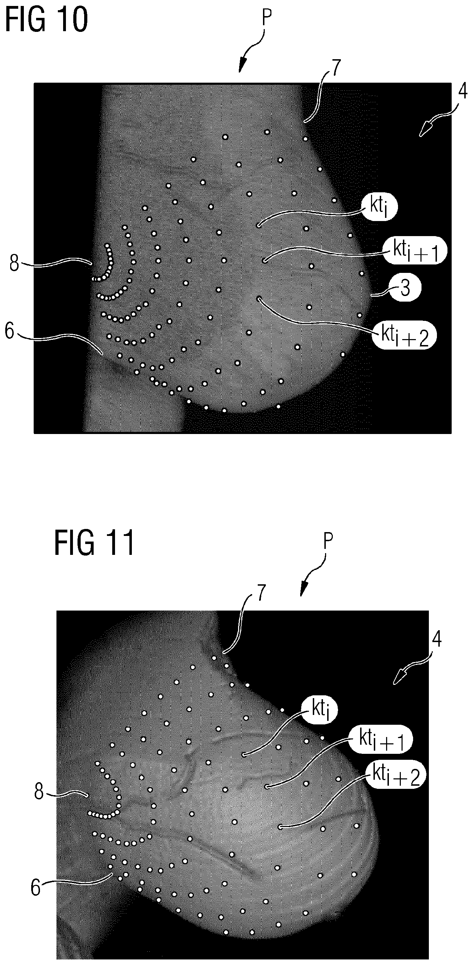

Mit Hilfe der so bestimmten Kurvenpunkte $\mathbf{k}_1$, ... , $\mathbf{k}_K$ bzw. der rotierten Brustebenen 22 können Konturenpunkte $\mathbf{kt}_1$, ... , $\mathbf{kt}_K$ in der Grenzfläche 9 der Bilddaten der ersten Form 1 durch Schnittbildung zwischen den rotierten Brustebenen 22 und der Grenzfläche 9 bestimmt werden. Dies wird beispielhaft in der Figur 10 gezeigt. In entsprechender Weise zeigt Figur 11 die Konturenpunkte $\mathbf{kt}_1$, ... , $\mathbf{kt}_K$ in der Grenzfläche 9 der Bilddaten der zweiten Form 2, welche wiederum durch Schnittbildung zwischen den rotierten Brustebenen 22 und der Grenzfläche 9 bestimmt wurden. Die Konturenpunkte $\mathbf{kt}_1$, ... , $\mathbf{kt}_K$ in der Grenzfläche 9 der Bilddaten der ersten Form 1 und den Bilddaten der zweiten Form 2 lassen sich nun einsetzen, um Zielobjekte 10, 11, die sich im Inneren der Brust 4 befinden, zu beschreiben. Dies kann beispielsweise durch eine räumliche Thin-Plate-Spline-Interpolation zwischen den Konturpunkten (d.h. beliebigen Punkten auf der Kontur) erzielt werden. Bei entsprechender Anwendung der Thin-Plate-Spline-Interpolation bei der Beschreibung der Zielobjekte 10 in den Bilddaten der ersten Form 1 auf die Konturen der Bilddaten der zweiten Form 2 ist es möglich, die Lage der Zielobjekte 11 in den Bilddaten der zweiten Form 2 zu bestimmen.

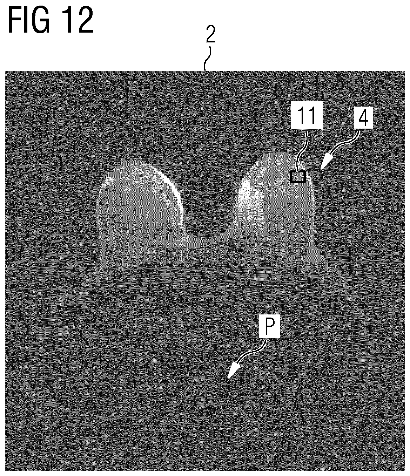

Die Figur 12 zeigt ein durch das erfindungsgemäße Verfahren bestimmte Zielobjekt 11 in den Bilddaten der zweiten Form 2. Wie der Figur 12 entnehmbar ist, weicht die Lage des bestimmten Zielobjekts 12 nur um etwa 10mm von der Läsion ab, welche in der Figur 12 als veränderte Einfärbung erkennbar ist. Eine derartige Abweichung bestätigt die Tauglichkeit des erfindungsgemäßen und automatisch ausführbaren Verfahrens für die medizinische Praxis.

Es wird abschließend noch einmal darauf hingewiesen, dass es sich bei den vorhergehend detailliert beschriebenen Verfahren und Bildbearbeitungsstationen lediglich um Ausführungsbeispiele handelt, welche vom Fachmann in verschiedenster Weise modifiziert werden können, ohne den Bereich der Erfindung zu verlassen. Insbesondere können z.B. die erfindungsgemäßen Verfahren nicht nur für den Körperbereich der Brust, sondern auch bei der radiologischen Bildgebung anderer Körperbereiche vorteilhaft eingesetzt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können.

**Patentansprüche**

1. Verfahren in der radiologischen Bildgebung zur Bestimmung einer zweiten Form von Bilddaten (2) aus einer ersten Form von Bilddaten (1) eines Untersuchungsobjekts (P), umfassend folgende Verfahrensschritte:

   - Bestimmung eines Satzes (**x**) einer Mehrzahl an Eingangsbildpunkten (**x**$_1$, ... , **x**$_K$) in den Bilddaten der ersten Form (1),
   - Prognostizierende Bestimmung eines Satzes an Zielformparametern (**y**) eines Zielformmodells mit einer Mehr-

zahl an Zielformparametern ($\mathbf{y}_1$, ..., $\mathbf{y}_L$) durch ein datengetriebenes Regressionsverfahren aus der Mehrzahl an Eingangsbildpunkten ($\mathbf{x}_1$, ... , $\mathbf{x}_K$), wobei die Mehrzahl an Zielformparametern ($\mathbf{y}_1$, ..., $\mathbf{y}_L$) kleiner ist als die Mehrzahl an Eingangsbildpunkten ($\mathbf{x}_1$, ... , $\mathbf{x}_K$),
- Bestimmung der zweiten Form von Bilddaten (2) aus dem Satz an Zielformparametern (**y**).

2. Verfahren nach Anspruch 1, **dadurch gekennzeichnet, dass** der Satz an Zielformparametern (**y**) eine Abweichung von einem standardisierten geometrischen Zielformmodell beschreibt.

3. Verfahren nach Anspruch 2, **dadurch gekennzeichnet, dass** das standardisierte geometrische Zielformmodell, bevorzugt durch Mittelwertbildung, aus gegebenen radiologischen Trainingsbilddatensätzen bestimmt wird.

4. Verfahren nach einem der Ansprüche 1 bis 3, **dadurch gekennzeichnet, dass** die Bestimmung eines Satzes (**x**) einer definierten Mehrzahl an Eingangsbildpunkten ($\mathbf{x}_1$, ... , $\mathbf{x}_K$) in den Bilddaten der ersten Form (1) die folgenden Verfahrensschritte umfasst:

- Segmentierung der ersten Form der Bilddaten (1), bevorzugt durch eine automatische Segmentierung, besonders bevorzugt durch eine automatische Segmentierung, die durch Schwellwertbildungs- und/oder Regionenwachstumsverfahrensschritte gekennzeichnet ist,
- Bestimmung einer Anzahl an anatomischen Landmarken (3, 6, 7) in der ersten Form der Bilddaten (1), bevorzugt mit automatischen Verfahren des maschinellen Lernens,
- Bestimmung einer Anzahl an Grenzflächen (9) in den segmentierten Bilddaten der ersten Form (1), bevorzugt nach einem Marching-Cube-Verfahren, wobei die geometrische Lage der Grenzflächen (9) durch die anatomischen Landmarken (3, 6, 7) festgelegt wird,
- Bestimmung der Eingangsbildpunkte ($\mathbf{x}_1$, ... , $\mathbf{x}_K$), so dass zumindest ein Teil der Eingangsbildpunkte ($\mathbf{x}_1$, ... , $\mathbf{x}_K$) auf den Grenzflächen (9) liegt.

5. Verfahren nach einem der Ansprüche 1 bis 4, **dadurch gekennzeichnet, dass** bei Körperbereichen des Untersuchungsobjekts (P), die als Paar an dem Untersuchungsobjekt (P) auftreten, das Verfahren für den ersten Teil des Paares auf den zweiten Teil des Paares anwendbar ist, indem vor der Bestimmung des Satzes an Eingangsbildpunkten ($\mathbf{x}_1$, ... , $\mathbf{x}_K$) die Bilddaten der ersten Form (1) des zweiten Teils des Paares an einer Spiegelachse gespiegelt werden und indem nach der Bestimmung der zweiten Form der Bilddaten (2) diese erneut an einer Spiegelachse gespiegelt werden.

6. Verfahren nach einem der Ansprüche 1 bis 5, **dadurch gekennzeichnet, dass** Körperbereiche des Untersuchungsobjekts (P) während der Aufnahme der ersten Form der Bilddaten (1) durch einen äußeren Mechanismus einer Verformung ausgesetzt sind und die zweite Form der Bilddaten (2) die Körperbereiche ohne diese Verformung darstellt.

7. Verfahren nach Anspruch 6, **dadurch gekennzeichnet, dass** die Bilddaten der ersten Form (1) durch ein Verfahren der Brusttomosynthese ermittelt werden und der der Verformung ausgesetzte Körperbereich eine Brust (4) umfasst.

8. Verfahren nach Anspruch 7, **dadurch gekennzeichnet, dass** der Satz an Eingangsbildpunkten ($\mathbf{x}_1$, ... , $\mathbf{x}_K$) so bestimmt wird, dass sie auf der Oberfläche der Brust (4) liegen und bevorzugt die Eingangsbildpunkte ($\mathbf{x}_1$, ... , $\mathbf{x}_K$) Gruppen zugeordnet sind, wobei die Eingangsbildpunkte ($\mathbf{x}_1$, ... , $\mathbf{x}_K$) einer Gruppe in etwa den gleichen Abstand von der Papilla (3) der Brust (4) aufweisen.

9. Verfahren nach einem der Ansprüche 1 bis 8, **dadurch gekennzeichnet, dass** das Regressionsverfahren als multiple, multi-variate Random-Forest-Regression ausgebildet ist.

10. Verfahren nach einem der Ansprüche 1 bis 9, **dadurch gekennzeichnet, dass** das Regressionsverfahren automatisch mit Verfahren des maschinellen Lernens aus radiologischen Trainingsbilddatensätzen abgeleitet ist.

11. Verfahren nach einem der Ansprüche 1 bis 10, **dadurch gekennzeichnet, dass** die Mehrzahl an Zielformparametern ($\mathbf{y}_1$, ..., $\mathbf{y}_L$) kleiner ist als die Mehrzahl an Eingangsbildpunkten ($\mathbf{x}_1$, ... , $\mathbf{x}_K$), wobei die Zielformparameter ($\mathbf{y}_1$, ..., $\mathbf{y}_L$) so gewählt sind, dass die kumulative Varianz des Zielformmodells (V) eine kumulative Varianz radiologischer Trainingsbilddatensätze mit wenigstens 80% abbildet.

12. Verfahren in der radiologischen Bildgebung zur Bestimmung der geometrischen Lage einer Anzahl von Zielobjekten (11) in einer zweiten Form von Bilddaten (2) aus der geometrischen Lage der Zielobjekte (10) in einer ersten Form von Bilddaten (1) eines Körperbereichs eines Untersuchungsobjekts (P), umfassend folgende Verfahrensschritte:

- Bestimmung der zweiten Form von Bilddaten (2) aus der ersten Form von Bilddaten (1) des Untersuchungsobjekts (P) mit einem Verfahren nach einem der Ansprüche 1 bis 11,
- Bestimmung einer Anzahl an Grenzflächen (9) des Körperbereichs in der ersten Form der Bilddaten (1) und in der zweiten Form der Bilddaten (2),
- Bestimmung einer Mehrzahl anatomischer Landmarken (3, 6, 7) in den Bilddaten der ersten Form (1) und in den Bilddaten der zweiten Form (2),
- Bestimmung einer geometrischen Kurve in der ersten Form der Bilddaten (1) und in der zweiten Form der Bilddaten (2), wobei die Kurven jeweils zumindest näherungsweise in der Grenzfläche (9) und durch die Mehrzahl anatomischer Landmarken (3, 6, 7) verlaufen,
- Bestimmung von Kurvenpunkten ($k_i$, $k_{i+1}$, $k_{i+2}$) in den geometrischen Kurven,
- Bestimmung einer Mehrzahl an Konturen in der Grenzfläche (9) der ersten Form der Bilddaten (1) und in der Grenzfläche (9) der zweiten Form der Bilddaten (2),
- Beschreibung der geometrischen Lage der Zielobjekte (10) in den Bilddaten der ersten Form (1) in Abhängigkeit von den Konturen der ersten Form der Bilddaten,
- Bestimmung der geometrischen Lage der Zielobjekte (11) in den Bilddaten der zweiten Form (2) in Abhängigkeit von den Konturen der zweiten Form der Bilddaten (2), unter Anwendung einer Interpolationen zwischen Konturen der ersten Form der Bilddaten (1) und Konturen der zweiten Form der Bilddaten (2).

13. Bildbearbeitungsstation (13) in der radiologischen Bildgebung zur Bestimmung einer zweiten Form von Bilddaten (2) aus einer ersten Form von Bilddaten (1) eines Untersuchungsobjekts (P) mit:

- einer Eingangsbildpunkteinrichtung (14) zur Bestimmung eines Satzes ($x$) einer Mehrzahl an Eingangsbildpunkten ($x_1$, ... , $x_K$) in den Bilddaten der ersten Form (1),
- einer Prognoseeinrichtung (15) zur Bestimmung eines Satzes an Zielformparametern ($y$) eines Zielformmodells mit einer Mehrzahl an Zielformparametern ($y_1$, ..., $y_L$) durch ein datengetriebenes Regressionsverfahren aus der Mehrzahl an Eingangsbildpunkten ($x_1$, ... , $x_K$), wobei die Mehrzahl an Zielformparametern ($y_1$, ..., $y_L$) kleiner ist als die Mehrzahl an Eingangsbildpunkten ($x_1$, ... , $x_K$) und
- einer Bilddatenbestimmungseinrichtung (16) zur Bestimmung der zweiten Form von Bilddaten (2) aus dem Satz an Zielformparametern ($y$).

14. Zielobjektbestimmungseinrichtung (20) in der radiologischen Bildgebung zur Bestimmung der geometrischen Lage einer Anzahl von Zielobjekten (11) in einer zweiten Form von Bilddaten (2) aus der geometrischen Lage der Zielobjekte (10) in einer ersten Form von Bilddaten (1) eines Körperbereichs eines Untersuchungsobjekts (P),

- wobei die Zielobjektbestimmungseinrichtung (20) eine Schnittstellenanordnung zur Übernahme von Bilddaten (1) des Untersuchungsobjekts (P) in einer ersten Form sowie von Daten betreffend Zielobjekten (10) in der ersten Form der Bilddaten (1) und zur Übernahme von Bilddaten in einer zweiten Form (2) aus einer Bildbearbeitungsstation (13) nach Anspruch 13 aufweist und
- wobei die Zielobjektbestimmungseinrichtung (20) ausgebildet ist, um folgende Schritte durchzuführen:

- Bestimmung einer Anzahl an Grenzflächen (9) des Körperbereichs in der ersten Form der Bilddaten (1) und in der zweiten Form der Bilddaten (2),
- Bestimmung einer Mehrzahl anatomischer Landmarken (3, 6, 7) in den Bilddaten der ersten Form (1) und in den Bilddaten der zweiten Form (2),
- Bestimmung einer geometrischen Kurve in der ersten Form der Bilddaten (1) und in der zweiten Form der Bilddaten (2), wobei die Kurven jeweils zumindest näherungsweise in der Grenzfläche (9) und durch die Mehrzahl anatomischer Landmarken (3, 6, 7) verlaufen,
- Bestimmung von Kurvenpunkten ($k_i$, $k_{i+1}$, $k_{i+2}$) in den geometrischen Kurven,
- Bestimmung einer Mehrzahl an Konturen in der Grenzfläche (9) der ersten Form der Bilddaten (1) und in der Grenzfläche (9) der zweiten Form der Bilddaten (2),
- Beschreibung der geometrischen Lage der Zielobjekte (10) in den Bilddaten der ersten Form (1) in Abhängigkeit von den Konturen der ersten Form der Bilddaten,
- Bestimmung der geometrischen Lage der Zielobjekte (11) in den Bilddaten der zweiten Form (2) in Abhängigkeit von den Konturen der zweiten Form der Bilddaten (2), unter Anwendung einer Interpolationen zwischen Konturen der ersten Form der Bilddaten (1) und Konturen der zweiten Form der Bilddaten (2).

**15.** Bildgebungseinrichtung mit einer Bildbearbeitungsstation (13) nach Anspruch 13 und/oder einer Zielobjektbestimmungseinrichtung (20) nach Anspruch 14.

**16.** Computerprogrammprodukt, welches direkt in einen Speicher einer programmierbaren Bildgebungseinrichtung oder Bildbearbeitungsstation (13) ladbar ist, mit Programmcode-Mitteln, um ein Verfahren nach einem der Ansprüche 1 bis 12 auszuführen, wenn das Programm in der Bildgebungseinrichtung oder Bildbearbeitungsstation (13) ausgeführt wird.

**Claims**

**1.** Method in radiological imaging for determining a second form of image data (2) from a first form of image data (1) of an examination object (P), consisting of the following method steps:

- The determination of a set ($x$) of a plurality of input pixels ($x_1, ... , x_K$) in the image data of the first form (1),
- The prognostic determination of a set of target form parameters ($y$) of a target form model with a plurality of target form parameters ($yi, ..., y_L$) by means of a data-driven regression method from the plurality of input pixels ($x_1, ... , x_K$), wherein the plurality of target form parameters ($yi, ..., y_L$) is smaller than the plurality of input pixels ($x_1, ... , x_K$),
- The determination of the second form of image data (2) from the set of target form parameters ($y$).

**2.** Method according to claim 1, **characterised in that** the set of target form parameters ($y$) describes a deviation from a standardised geometric target form model.

**3.** Method according to claim 2, **characterised in that** the standardised geometric target form model is determined from given radiological training image data sets, preferably by means of averaging.

**4.** Method according to one of the claims 1 to 3, **characterised in that** the determination of a set ($x$) of a defined plurality of input pixels ($x_1, ... , x_K$) in the image data of the first form (1) consists of the following method steps:

- The segmentation of the first form of the image data (1), preferably by means of automatic segmentation, particularly preferably by means of automatic segmentation which is **characterised by** thresholding and/or regional growing method steps.
- The determination of a number of anatomical landmarks (3, 6, 7) in the first form of the image data (1), preferably with automatic methods of machine learning,
- The determination of a number of interfaces (9) in the segmented image data of the first form (1), preferably according to a marching cube method, wherein the geometric position of the interfaces (9) is established by means of the anatomical landmarks (3, 6, 7),
- The determination of the input pixels ($x_1, ... , x_K$) so that at least some of the input pixels ($x_1, ... , x_K$) lie on the interfaces (9).

**5.** Method according to one of the claims 1 to 4, **characterised in that** in areas of the body of the examination object (P) which occur in pairs on the examination object (P), the method for the first part of the pair is applicable to the second part of the pair, with the image data of the first form (1) of the second part of the pair being mirrored in a mirror axis before the determination of the set of input pixels ($x_1, ... , x_K$) and with this being mirrored again in a mirror axis after the determination of the second form of the image data (2).

**6.** Method according to one of the claims 1 to 5, **characterised in that** areas of the body of the examination object (P) are subject to deformation by an external mechanism during the recording of the first form of image data (1) and the second form of image data (2) represents the areas of the body without this deformation.

**7.** Method according to claim 6, **characterised in that** the image data of the first form (1) is ascertained by means of a method of breast tomosynthesis and the area of the body subject to deformation consists of a breast (4).

**8.** Method according to claim 7, **characterised in that** the set of input pixels ($x_1, ... , x_K$) is determined in such a way that they lie on the surface of the breast (4) and preferably the input pixels ($x_1, ... , x_K$) are assigned to groups, wherein the input pixels ($x_1, ... , x_K$) of a group are located at approximately the same distance from the papilla (3) of the breast (4).

9. Method according to one of the claims 1 to 8, **characterised in that** the regression method is embodied as multiple, multivariate random forest regression.

10. Method according to one of the claims 1 to 9, **characterised in that** the regression method is automatically derived from radiological training image data sets using methods of machine learning.

11. Method according to one of the claims 1 to 10, **characterised in that** the plurality of target form parameters ($\mathbf{y}_1$, ..., $\mathbf{y}_L$) is significantly smaller than the plurality of input pixels ($\mathbf{x}_1$, ... , $\mathbf{x}_K$), wherein the target form parameters ($\mathbf{y}_1$, ..., $\mathbf{y}_L$) are selected in such a way that the cumulative variance of the target form model (V) reflects a cumulative variance of radiological training image data sets by at least 80%.

12. Method in radiological imaging for determining the geometric position of a number of target objects (11) in a second form of image data (2) from the geometric position of the target objects (10) in a first form of image data (1) of an area of the body of an examination object (P), consisting of the following method steps:

   - The determination of the second form of image data (2) from the first form of the image data (1) of the examination object (P), using a method according to one of the claims 1 to 11,
   - The determination of a number of interfaces (9) of the area of the body in the first form of the image data (1) and in the second form of the image data (2),
   - The determination of a plurality of anatomical landmarks (3, 6, 7) in the image data of the first form (1) and in the image data of the second form (2),
   - The determination of a geometric curve in the first form of the image data (1) and in the second form of the image data (2), wherein each of the curves run at least approximately in the interface (9) and through the plurality of anatomical landmarks (3, 6, 7),
   - The determination of curve points ($\mathbf{k}_i$, $\mathbf{k}_{i+1}$, $\mathbf{k}_{i+2}$) in the geometric curves,
   - The determination of a plurality of contours in the interface (9) of the first form of the image data (1) and in the interface (9) of the second form of the image data (2),
   - The description of the geometric position of the target objects (10) in the image data of the first form (1) as a function of the contours of the first form of the image data,
   - The determination of the geometric position of the target objects (11) in the image data of the second form (2) as a function of the contours of the second form of the image data (2), using interpolation between contours of the first form of the image data (1) and contours of the second form of the image data (2).

13. Image processing workstation (13) in radiological imaging for determining a second form of image data (2) from a first form of image data (1) of an examination object (P) with:

   - An input pixel device (14) for determining a set ($\mathbf{x}$) of a plurality of input pixels ($\mathbf{x}_1$, ... , $\mathbf{x}_K$) in the image data of the first form (1),
   - A prognostic device (15) for determining a set of target form parameters ($\mathbf{y}$) of a target form model with a plurality of target form parameters ($\mathbf{y}_1$, ..., $\mathbf{y}_L$) by means of a data-driven regression method from the plurality of input pixels ($\mathbf{x}_1$, ... , $\mathbf{x}_K$), wherein the plurality of target form parameters ($\mathbf{y}_1$, ..., $\mathbf{y}_L$) is smaller than the plurality of input pixels ($\mathbf{x}_1$, ... , $\mathbf{x}_K$) and
   - An image data determination device (16) for determining the second form of image data (2) from the set of target form parameters ($\mathbf{y}$).

14. Target object determination device (20) in radiological imaging for determining the geometric position of a number of target objects (11) in a second form of image data (2) from the geometric position of the target objects (10) in a first form of image data (1) of an area of the body of an examination object (P),

   - wherein the target object determination device (20) has an interface arrangement for the acceptance of image data (1) of the examination object (P) in a first form and of data concerning target objects (10) in the first form of the image data (1) and for the acceptance of image data in a second form (2) from an image processing workstation (13) according to claim 13 and
   - wherein the target object determination device (20) is embodied in order to carry out the following steps:

      - The determination of a number of interfaces (9) of the area of the body in the first form of the image data (1) and in the second form of the image data (2),
      - The determination of a plurality of anatomical landmarks (3, 6, 7) in the image data of the first form (1) and in the image data of the second form (2),
- The determination of a geometric curve in the first form of the image data (1) and in the second form of the image data (2), wherein the curves each run at least approximately in the interface (9) and through the plurality of anatomical landmarks (3, 6, 7),
- The determination of curve points ($k_i$, $k_{i+1}$, $k_{i+2}$) in the geometric curves,
- The determination of a plurality of contours in the interface (9) of the first form of the image data (1) and in the interface (9) of the second form of the image data (2),
- The description of the geometric position of the target objects (10) in the image data of the first form (1) as a function of the contours of the first form of the image data,
- The determination of the geometric position of the target objects (11) in the image data of the second form (2) as a function of the contours of the second form of the image data (2), using interpolation between contours of the first form of the image data (1) and contours of the second form of the image data (2).

15. Imaging device with an image processing workstation (13) according to claim 13 and/or a target object determination device (20) according to claim 14.

16. Computer program product which can be loaded directly into a memory of a programmable imaging device and/or image processing workstation (13), having program code means, in order to execute a method according to one of the claims 1 to 12, when the program is executed in the imaging device and/or image processing workstation (13).

**Revendications**

1. Procédé dans l'imagerie radiologique de détermination d'une deuxième forme de données (2) d'image à partir d'une première forme de données (1) d'image d'un objet (P) à examiner, comprenant les stades de procédé suivants :

   - détermination d'un jeu ($\mathbf{x}$) d'une pluralité de pixels ($\mathbf{x}_1$, ..., $\mathbf{x}_K$) d'entrée dans les données d'image de la première forme (1),
   - détermination donnant un pronostic d'un jeu de paramètres ($\mathbf{y}$) de forme cible d'un modèle de forme cible ayant une pluralité de paramètres ($\mathbf{y}_1$, ..., $\mathbf{y}_L$) de forme cible par un procédé de régression par les données à partir de la pluralité de pixels ($\mathbf{x}_1$, ..., $\mathbf{x}_K$) d'entrée, la pluralité de paramètres ($\mathbf{y}_1$, ..., $\mathbf{y}_L$) de forme cible étant plus petite que la pluralité de pixels ($\mathbf{x}_1$, ..., $\mathbf{x}_K$) d'entrée,
   - détermination de la deuxième forme de données (2) d'image à partir du jeu de paramètres ($\mathbf{y}$) de forme cible.

2. Procédé suivant la revendication 1, **caractérisé en ce que** le jeu de paramètres ($\mathbf{y_1}$, ..., $\mathbf{y}_L$) de forme cible décrit un écart à un modèle de forme cible géométrique normalisé.

3. Procédé suivant la revendication 2, **caractérisé en ce que** l'on détermine le modèle de forme cible géométrique normalisé, de préférence par formation de moyenne, à partir de jeux de données d'image d'apprentissage radiologiques donnés.

4. Procédé suivant l'une des revendications 1 à 3, **caractérisé en ce que** la détermination d'un jeu ($\mathbf{x}$) d'une pluralité définie de pixels ($\mathbf{x}_1$, ..., $\mathbf{x}_K$) d'entrée dans les données d'image de la première forme (1) comprend les stades de procédé suivants :

   - segmentation de la première forme de données (1) d'image, de préférence par une segmentation automatique, d'une manière particulièrement préférée par une segmentation automatique, qui est **caractérisée par** des stades de procédé de formation de valeurs de seuil et/ou de croissance de région,
   - détermination d'un nombre de repères (3, 6, 7) anatomique dans la première forme de données (1) d'image, de préférence par un procédé automatique de l'apprentissage artificiel,
   - détermination d'un nombre de surfaces (9) limites dans les données d'image segmentées de la première forme (1), de préférence par un procédé marching-cube, la position géométrique des surfaces (9) limites étant fixée par les repères (3, 6, 7) anatomiques,
   - détermination des pixels ($\mathbf{x}_1$, ..., $\mathbf{x}_K$) d'entrée de manière à ce qu'au moins une partie des pixels ($\mathbf{x}_1$, ..., $\mathbf{x}_K$) d'entrée soit sur les surfaces (9) limites.

5. Procédé suivant l'une des revendications 1 à 4, **caractérisé en ce que**, pour des parties du corps de l'objet (P) à examiner, qui apparaissent en paire sur l'objet (P) à examiner, le procédé peut, pour la première partie de la paire, être appliqué à la deuxième partie de la paire, en prenant la symétrie, avant la détermination du jeu de pixels ($x_1$, ..., $x_K$) d'entrée, des données d'image de la première forme (1) de la deuxième partie de la paire par rapport à un axe de symétrie, et en prenant à nouveau la symétrie par rapport à un axe de symétrie, après la détermination de la deuxième forme des données (2) d'image.

**6.** Procédé suivant l'une des revendications 1 à 5, **caractérisé en ce que** les parties du corps de l'objet (P) à examiner pendant l'enregistrement de la première forme des données (1) d'image sont soumises à une déformation par un mécanisme extérieur et la deuxième forme des données (2) d'image représente les parties du corps sans cette déformation.

**7.** Procédé suivant la revendication 6, **caractérisé en ce que** l'on détermine les données d'image de la première forme (1) par un procédé de tomosynthèse du sein et **en ce que** la partie du corps soumise à la déformation comprend un sein (4).

**8.** Procédé suivant la revendication 7, **caractérisé en ce que** l'on détermine le jeu de pixels ($x_1$, ..., $x_K$) d'entrée de manière à ce qu'il se trouve à la surface du sein (4) et, de préférence les pixels ($x_1$, ..., $x_K$) d'entrée sont affectés à des groupes, les pixels ($x_1$, ..., $x_K$) d'entrée d'un groupe étant à peu près à la même distance de la papille (3) du sein (4).

**9.** Procédé suivant l'une des revendications 1 à 8, **caractérisé en ce que** le procédé de régression est constitué sous la forme d'une régression random-forest multivariable.

**10.** Procédé suivant l'une des revendications 1 à 9, **caractérisé en ce que** le procédé de régression est déduit automatiquement par un procédé de l'apprentissage de jeux de données d'image d'apprentissage radiologiques.

**11.** Procédé suivant l'une des revendications 1 à 10, **caractérisé en ce que** la pluralité de paramètres ($y_1$, ..., $y_L$) de forme cible est plus petite que la pluralité de pixels ($x_1$, ..., $x_K$) d'entrée, les paramètres ($y_1$, ..., $y_L$) de forme cible étant choisis de manière à ce que la variance cumulée du modèle (V) de forme cible reproduise, à au moins 80%, une variance cumulée de jeux de données d'image d'apprentissage radiologiques.

**12.** Procédé dans l'imagerie radiologique de détermination de la position géométrique d'un certain nombre d'objets (11) cibles dans une deuxième forme de données (2) d'image à partir de la position géométrique des objets (10) cibles dans une première forme de données (1) d'image d'une partie du corps d'un objet (P) à examiner, comprenant les stades de procédé suivants :

  - détermination de la deuxième forme de données (2) d'image à partir de la première forme de données (1) d'image de l'objet (P) à examiner par un procédé suivant l'une des revendications 1 à 11,
  - détermination d'un certain nombre de surfaces (9) limites de la partie du corps dans la première forme de données (1) d'image et dans la deuxième forme de données (2) d'image,
  - détermination d'une pluralité de repères (3, 6, 7) anatomiques dans les données d'image de la première forme (1) et dans les données d'image de la deuxième forme (2),
  - détermination d'un courbe géométrique dans la première forme des données (1) d'image et dans la deuxième forme des données (2) d'image, les courbes s'étendant respectivement, au moins approximativement, dans la surface (9) limite et passant par la pluralité de repères (3, 6, 7) anatomiques,
  - détermination de points ($k_i$, $k_{i+1}$, $k_{i+2}$) de la courbe géométrique,
  - détermination d'une pluralité de contours dans la surface (9) limite de la première forme des données (1) d'image et dans la surface (9) limite de la deuxième forme des données (2) d'image,
  - description de la position géométrique des objets (10) cibles dans les données d'image de la première forme (1) en fonction des contours de la première forme des données d'image,
  - détermination de la position géométrique des objets (11) cibles dans les données d'image de la deuxième forme (2) en fonction des contours de la deuxième forme des données (2) d'image, en utilisant une interpolation entre des contours de la première forme des données (1) d'image et des contours de la deuxième forme des données (2) d'image.

**13.** Poste (13) de traitement d'image dans l'imagerie radiologique pour déterminer une deuxième forme de données (2) d'image à partir d'une première forme de données (1) d'image d'un objet (P) à examiner, comprenant :

  - un dispositif (14) de pixels d'entrée pour déterminer un jeu ($x$) d'une pluralité de pixels ($x_1$, ..., $x_K$) d'entrée dans les données d'image de la première forme (1),
- un dispositif (15) de pronostic pour déterminer un jeu de paramètres (**y**) de forme cible d'un modèle de forme cible, comprenant une pluralité de paramètres (**y**$_1$, ..., **y**$_L$) de forme cible par un procédé de régression par données à partir de la pluralité de pixels (**x**$_1$, ..., **x**$_K$) d'entrée, la pluralité de paramètres (**y**$_1$, ..., **y**$_L$) de forme cible étant plus petite que la pluralité de pixels (**x**$_1$, ..., **x**$_K$) d'entrée et
- un dispositif (16) de détermination de données d'image pour déterminer la deuxième forme de données (2) d'image à partir du jeu de paramètres (**y**) de forme cible.

**14.** Dispositif (20) de détermination d'objets cibles dans l'imagerie radiologique pour déterminer la position géométrique d'un certain nombre d'objets (11) cibles dans une deuxième forme de données (2) d'image à partir de la position géométrique des objets (10) cibles dans une première forme de données (1) d'image d'une partie du corps d'un objet (P) à examiner,

- dans lequel le dispositif (20) de détermination d'objets cibles a un système d'interface pour la prise en charge de données (1) d'image de l'objet (P) à examiner dans une première forme, ainsi que de données concernant des objets (10) cibles dans la première forme des données (1) d'image, et pour la prise en charge de données d'image dans une deuxième forme (2) à partir d'un poste (13) de traitement d'image suivant la revendication 13 et
- dans lequel le dispositif (20) de détermination d'objets cibles est constitué pour effectuer les stades suivants :

- détermination d'un certain nombre de surfaces (9) limites de la partie du corps dans la première forme des données (1) d'image et dans la deuxième forme des données (2) d'image,
- détermination d'une pluralité de repères (3, 6, 7) anatomiques dans les données d'image de la première forme (1) et dans les données d'image de la deuxième forme (2),
- détermination d'une courbe géométrique dans la première forme des données (1) d'image et dans la deuxième forme des données (2) d'image, les courbes s'étendant, au moins à peu près, dans la surface (9) limite et passant par la pluralité de repères (3, 6, 7) anatomiques,
- détermination de points (**k**$_i$, **k**$_{i+1}$, **k**$_{i+2}$) dans les courbes géométriques,
- détermination d'une pluralité de contours dans la surfacess (9) limitess de la première forme des données (1) d'image et dans la deuxième surface (9) limite de la deuxième forme des données (2) d'image,
- description de la position géométrique des objets (10) cibles dans les données d'image de la première forme (1) en fonction des contours de la première forme des données d'image,
- détermination de la position géométrique des objets (11) cibles dans les données d'image de la deuxième forme (2) en fonction des contours de la deuxième forme des données (2) d'image, en utilisant une interpolation entre des contours de la première forme des données (1) d'image et des contours de la deuxième forme des données (2) d'image.

**15.** Dispositif d'imagerie ayant un poste (13) de traitement de données suivant la revendication 13 et/ou dispositif (20) de détermination d'objets cibles suivant la revendication 14.

**16.** Produit de programme d'ordinateur, qui peut être chargé directement dans une mémoire d'un dispositif d'imagerie programmable ou dans un poste (13) de traitement d'image programmable, comprenant des moyens de code de programme pour effectuer un procédé suivant l'une des revendications 1 à 12, lorsque le programme est réalisé dans le dispositif d'imagerie ou dans le poste (13) de traitement d'image.

FIG 1

$$x = (x_1, ..., x_K)^T \qquad y = (y_1, ..., y_L)^T$$

FIG 2

FIG 3

# FIG 4

EP 2 634 748 B1

FIG 5

FIG 6

FIG 7

## FIG 8

## FIG 9

FIG 10

FIG 11

## FIG 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **G. SCHIE et al.** Correlating locations in ipsilateral breast tomosynthesis views using an analytical hemispherical compression model. *Phys. Med. Biol.,* 2011, 56

- **STELIOS K. KYRIACOU et al.** Nonlinear Elastic Registration of Brain Images with Tumor Pathology Using a Biomechanical Model. *IEEE Transactions on Medical Imaging,* Juli 1999, vol. 18 (7